# EUROPEAN PATENT APPLICATION

(11) **EP 2 343 321 A1**
(43) Date of publication of application: **13.07.2011**
(21) Application number: 10182217.9
(22) Date of filing: 12.11.2004
(51) Int. Cl.: C07K 16/30, A61K 39/395, G01N 33/574

(54) **Human antibodies specific for carcinoma**

(30) Priority: 12.11.2003 US 519550 P
(62) Divisional of application: 04821664.2
(71) Applicant: Patrys Limited, Melbourne, Victoria 3000 (AU)
(72) Inventor: Vollmers, Heinz Peter, 97078 Würzburg (DE); Müller-Hermelink, Hans Konrad, 97082 Würzburg (DE)
(74) Representative: Cohausz & Florack

(57) **Abstract**

The present invention features antibodies, which may be isolated from healthy donors, that are specific for carcinoma and their use in the treatment and diagnosis of carcinoma.

## Description

### Background of the Invention

The present invention is related to the field of cancer diagnosis and treatment and, more specifically, to the identification, from healthy donors, of polypeptides, such as antibodies, useful in the diagnosis, detection, monitoring, and treatment of neoplasms in a mammal, e.g., a human.

In the United States well over one million individuals are diagnosed with cancer each year. Although recent advances in the medical field have significantly improved the rate of survival among cancer patients, a large number of cancer-related deaths still could be prevented by the early diagnosis of the tumor. Accordingly, at the time of initial diagnosis, an alarming number of patients have already reached late stages of the disease.

With respect to colorectal cancer, the prognosis is usually poor in 50% of all cases because the tumor is often undetected until the disease has spread and reached a terminal stage. Clearly, there is a need for the early and improved detection and treatment of neoplasms (e.g., stomach adenocarcinoma, colorectal adenocarcinoma, lung adenocarcinoma, adenocarcinoma of the pancreas), as this would increase the chance of treating the neoplasm and, thereby, lead to an improved prognosis for long-term survival.

### Summary of the Invention

Using cells derived from healthy donors, we have discovered a class of polypeptides which react with epitopes specific for neoplastic cells. These polypeptides are not only excellent diagnostic tools, but can also induce apoptosis of the neoplastic cells to which they bind. This latter characteristic results in a treatment for neoplastic diseases that lacks the side-effects of many existing therapeutics. In addition, our finding that healthy donors can harbor cells that express neoplasm-specific polypeptides provides a novel use of cells and tissue derived from healthy donors in methods of identifying polypeptides that can be used in the diagnosis and treatment of neoplasms, such as cancers.

Accordingly, in the first aspect, the invention features a method for identifying an isolated polypeptide, e.g., an antibody such as a monoclonal antibody, that specifically binds to a neoplastic cell and does not bind to a non-neoplastic cell. This method includes the steps of (1) providing an isolated cell derived from a healthy donor, for example, a human, (2) isolating a polypeptide produced by the cell, and (3) determining whether the polypeptide specifically binds to a neoplastic cell and does not bind to a non-neoplastic cell.

In a desirable embodiment of the first aspect, the neoplastic cell is not a neuroblastoma cell. In other desirable embodiments, step (1) also involves immortalizing the isolated cell, for example, by fusing the isolated cell with a myeloma or heteromyeloma cell, and step (3) involves determining whether contacting the neoplastic and the non-neoplastic cell with the polypeptide induces apoptosis in the neoplastic cell and not in the non-neoplastic cell. In a further desirable embodiment of the first aspect, step (3) involves determining whether contacting the neoplastic and the non-neoplastic cell with the polypeptide reduces proliferation of the neoplastic cell and not of the non-neoplastic cell. For example, the neoplastic cell may be a carcinoma, such as an adenocarcinoma of the colon, diffuse-type stomach carcinoma, adenocarcinoma of the pancreas, or adenocarcinoma of the lung. In addition, the antibody may be an IgM or a monoclonal antibody. Furthermore, the isolated cell of step (1) may be a lymphocyte, e.g., one derived from a spleen, a lymph node, or from blood.

In a second aspect, the invention features an isolated cell expressing a polypeptide identified using the method of the first aspect of the invention.

The third aspect of the invention feature a purified polypeptide including the amino acid sequence of SEQ ID NO:1 or 3; the fourth aspect of the invention features a purified polypeptide including the amino acid sequence of SEQ ID NOS:1 and 3; the fifth aspect of the invention features a purified polypeptide including the amino acid sequence of SEQ ID NO:5 or 7; the sixth aspect of the invention features a purified polypeptide including the amino acid sequence of SEQ ID NOS:5 and 7; the seventh aspect of the invention features a purified polypeptide including amino acids 31-35, 50-66, and 99-107 of SEQ ID NO:1 or amino acids 23-33, 49-55, and 88-99 of SEQ ID NO:3; and the eighth aspect of the invention features a purified polypeptide including amino acids 31-35, 50-66, and 99-108 of SEQ ID NO:5 or amino acids 23-36, 52-58, and 91-101 of SEQ ID NO:7. In desirable embodiments of the third through eighth aspects, the polypeptide is an antibody, e.g., a monoclonal antibody, or a functional fragment thereof. For example, the functional fragment may be selected from the group consisting of V_{L}, V_{H}, F_{V}, F_{C}, Fab, Fab', and F(ab')₂. In addition, the functional fragment may include amino acids 31-35, 50-66, and 99-107 of SEQ ID NO:1, amino acids 23-33, 49-55, and 88-99 of SEQ ID NO:3, amino acids 31-35, 50-66, and 99-108 of SEQ ID NO:5 or amino acids 23-36, 52-58, and 91-101 of SEQ ID NO:7 and, desirably, is a V_{L} chain of an antibody. Furthermore, the functional fragment may specifically bind to an adenocarcinoma of the colon, a diffuse-type stomach carcinoma, an adenocarcinoma of the pancrease, and/or an adenocarcinoma of the lung, and not to a non-neoplastic cell of the same tissue type.

In the ninth aspect, the invention features a purified polypeptide that specifically binds to an adenocarcinoma of the colon, a diffuse-type stomach carcinoma, an adenocarcinoma of the pancreas, and an adenocarcinoma of the lung, and not to non-neoplastic cells of the same tissue type. In addition, the polypeptide of the ninth aspect includes an amino acid sequence that is substantially, e.g., at least 80%, identical to the full-length sequence of SEQ ID NO:1 and/or SEQ ID NO:3. For example, the polypeptide may be one encoded by a nucleic acid sequence that is substantially identical to the full-length nucleic acid sequence of SEQ ID NO:2 or 4. In a desirable embodiment of the ninth aspect of the invention, the polypeptide specifically binds to EPLC-272H (DSMZ (Deutsche Sammlung von Mikroorganismen und Zelllculturen GmbH; German Collection of Microorganisms and Cell Cultures) Accession Number ACC 383), Colo-699 (DSMZ Accession Number ACC 196), CACO-2 (DSMZ Accession Number ACC169, ATCC (American Type Culture Collection) Accession Number HTB-37), Colo-206F (DSMZ Accession Number ACC 21), 23132/87 (DSMZ Accession Number ACC 201), ASPC-1 (ATCC Accession Number CRL-1682), DU-145 (DSMZ Accession Number ACC 261, ATCC Accession Number HTB-81), and BM1604 (DSMZ Accession Number ACC 298) cells.

In other desirable embodiments of the ninth aspect of the invention, the polypeptide induces apoptosis in the neoplastic cell, but does not induce apoptosis in the non-neoplastic cell or the polypeptide decreases proliferation of the neoplastic cell, but does not decrease proliferation of the non-neoplastic cell.

The tenth aspect of the invention features a purified polypeptide that specifically binds to an adenocarcinoma of the colon, a diffuse-type stomach carcinoma, an adenocarcinoma of the pancreas, and an adenocarcinoma of the lung, and not to non-neoplastic cells of the same tissue type. In addition, the polypeptide of the tenth aspect includes an amino acid sequence that is substantially, e.g., at least 80%, identical to the full-length sequence of SEQ ID NO:5 and/or SEQ ID NO:7. For example, the polypeptide may be one encoded by a nucleic acid sequence that is substantially identical to the full-length nucleic acid sequence of SEQ ID NO:6 or 8. In a desirable embodiment of the tenth aspect of the invention, the polypeptide specifically binds to Colo-699 (DSMZ Accession Number ACC 196), CACO-2 (DSMZ Accession Number ACC169, ATCC Accession Number HTB-37), 23132/87 (DSMZ Accession Number ACC 201), DU-145 (DSMZ Accession Number ACC 261, ATCC Accession Number HTB-81), and BM1604 (DSMZ Accession Number ACC 298) cells.

In other desirable embodiments of the tenth aspect of the invention, the polypeptide induces apoptosis in the neoplastic cell, but does not induce apoptosis in the non-neoplastic cell or the polypeptide decreases proliferation of the neoplastic cell, but does not decrease proliferation of the non-neoplastic cell.

In additional desirable embodiments of the third through tenth aspects of the invention, the polypeptide is also produced by the NORM-1 cell line having DSMZ Deposit Accession No. DSM ACC2624, or by the NORM-2 cell line having DSMZ Deposit Accession No. DSM ACC2626.

The eleventh aspect of the invention features an isolated nucleic acid molecule including the sequence of SEQ ID NO:2; the twelfth aspect of the invention features an isolated nucleic acid molecule including the sequence of SEQ ID NO:4; the thirteenth aspect of the invention features an isolated nucleic acid molecule including the sequence of SEQ ID NO: 6; the fourteenth aspect of the invention features an isolated nucleic acid molecule including the sequence of SEQ ID NO: 8; the fifteenth aspect of the invention features an isolated nucleic acid molecule including nucleic acids 91-105, 148-198, and 295-321 of SEQ ID NO:2 or nucleic acids 67-99, 145-165, and 262-297 of SEQ ID NO:4; and the sixteenth aspect of the invention features an isolated nucleic acid molecule including nucleic acids 91-105, 148-198, and 295-324 of SEQ ID NO:6 or nucleic acids 67-108, 154-174, and 271-303 of SEQ ID NO:8. In desirable embodiments, the invention features nucleic acid molecules that hybridize to the sequence of SEQ ID NO:2, 4, 6, or 8, or a functional fragment thereof, under highly stringent conditions. In other desirable embodiments, the nucleic acid molecule of the fifteenth aspect may include nucleic acids 327-357 of SEQ ID NO:2 or nucleic acids 291-300 of SEQ ID NO:4. In the seventeenth aspect, the invention features a vector that includes the nucleic acid sequence of any one of the eleventh through sixteenth aspects of the invention. The eighteenth aspect of the invention features an isolated cell that includes the vector of the seventeenth aspect.

In the nineteenth aspect, the invention features an isolated cell that expresses the polypeptide of any one of the third through eighth aspects of the invention. In desirable embodiments of the nineteenth aspect, the isolated cell is a mammalian cell, such as a human cell. In other desirable embodiments of the nineteenth aspect, the polypeptide expressed by the cell is an antibody, e.g., an IgM antibody or a monoclonal antibody.

In the twentieth aspect, the invention features a method of producing the purified polypeptide of any one of the third through eighth aspects of the invention. This method involves contacting a cell with the vector of the seventeenth aspect of the invention and isolating the polypeptide expressed by the cell.

In the twenty-first aspect, the invention features use of the purified polypeptide of any one of aspects three through eight of the invention in a method of diagnosing a neoplasm in a mammal. This method involves the steps of, (a) contacting a cell or tissue sample derived from the mammal with the purified polypeptide of any one of aspects three through eight of the invention, and (b) detecting whether the purified polypeptide binds to the cell, where binding of the purified polypeptide to the cell is indicative of the mammal having a neoplasm. In a desirable embodiment of the twenty-first aspect of the invention, the mammal is a human. In another desirable embodiment of the twenty-first aspect, the polypeptide is an antibody, e.g., a monoclonal antibody. In further desirable embodiments of the twenty-first aspect of the invention, the polypeptide is conjugated to a detectable agent selected from the group consisting of a radionuclide, a fluorescent marker, an enzyme, a cytotoxin, a cytokine, and a growth inhibitor. In addition, the detectable agent may be capable of inducing apoptosis of the cell. Furthermore, the polypeptide used in the twenty-first aspect of the invention may be conjugated to a protein purification tag, such as a cleavable protein purification tag.

In the twenty-second aspect, the invention features use of the purified polypeptide of any one of aspects three through eight of the invention in a method of treating a proliferative disorder in a mammal. This method involves the step of contacting a cell with the purified polypeptide of any one of aspects three through eight of the invention, where binding of the purified polypeptide to the cell results in a reduction in proliferation of the cell. In a desirable embodiment of the twenty-second aspect of the invention, the mammal is a human. In another desirable embodiment of the twenty-second aspect, the polypeptide is an antibody. In a further desirable embodiment of the twenty-second aspect, the polypeptide is conjugated to a detectable agent selected from the group consisting of a radionuclide, a fluorescent marker, an enzyme, a cytotoxin, a cytokine, and a growth inhibitor. In addition, this detectable agent may be capable of inhibiting cell proliferation of the cell. In other desirable embodiments, the polypeptide of the twenty-second aspect of the invention is conjugated to a protein purification tag, such as a cleavable protein purification tag.

In the twenty-third aspect, the invention features a medicament including the purified polypeptide of any one of aspects three through eight of the invention in a pharmaceutically acceptable carrier and in the twenty-fourth aspect, the invention features a diagnostic agent including the purified polypeptide of any one of aspects three through eight of the invention.

In other desirable aspects, the invention features an antibody producing cell line having DSMZ accession number DSM ACC2624 or DSM ACC2626, as well as the antibodies produced by these cell lines.

In additional desirable embodiments of the first aspect of the invention, the isolated polypeptide does not specifically bind to cells of the following neuroblastoma cell lines: LA-N-1 and LA-N-5 (Juhl et al., Mol. Immunol. 27:957-964, 1990); SK-N-SH (ATCC Accession No. HTB-11); NMB-7 (Cheung et al., Cancer Res. 45:2642-2649, 1985); IMR-32 (ATCC Accession No. CCL-127); SH-SY5Y (Melino and Finazzi-Agro, in Human Neuroblastoma: Recent Advances in Clinical and Genetic Analysis, eds. Schwab, Tonini, and Benard, Harwood, Chur, Switzerland, pp. 55-71, 1993); or SK-N-MC (ATCC Accession No. HTB-10).

### Definitions

By "detectable agent" is meant a compound that is linked to a diagnostic agent to facilitate detection. Such a "detectable agent" may be covalently or non-covalently linked to a diagnostic agent. In addition, the linkage may be direct or indirect. Examples of "detectable agents" include, protein purification tags, cytotoxins, enzymes, paramagnetic labels, enzyme substrates, co-factors, enzymatic inhibitors, dyes, radionuclides, chemiluminescent labels, fluorescent markers, growth inhibitors, cytokine, antibodies, and biotin.

By a "diagnostic agent" is meant a compound that may be used to detect a neoplastic cell by employing any one of the assays described herein as well as any other method that is standard in the art. A diagnostic agent may include, for example, an antibody which specifically binds to at least one of the following cells: EPLC-272H (DSMZ Accession No. ACC 383), Colo-699 (DSMZ Accession No. ACC 196), CACO-2 (ATCC Accession No. HBT-37; DSMZ Accession No. ACC 169), Colo-206F (DSMZ Accession No. ACC 21), 23132/87 (DSMZ Accession No. ACC 201), ASPC-1 (ATCC Accession No. CRL-1682), DU-145 (DSMZ Accession No. ACC 261, ATCC Accession No. HTB-81), and BM10604 (DSMZ Accession No. ACC 298), but not to non-neoplastic cells. In addition, a diagnostic agent may specifically bind to an adenocarcinoma of the colon, a diffuse-type stomach carcinoma, an adenocarcinoma of the pancrease, and/or an adenocarcinoma of the lung, and not to a non-neoplastic cell of the same tissue type. Furthermore, a "diagnostic agent" may inhibit cell proliferation, induce apoptosis, or both only when it is bound to a neoplastic cell, but not a non-neoplastic cell.

Examples of neoplastic cells that may be detected with such a "diagnostic agent" include stomach adenocarcinoma, colorectal adenocarcinoma, squamous cell lung carcinoma, lung adenocarcinoma, adenocarcinoma of the pancreas, and adenocarcinoma of the prostate. Moreover, a "diagnostic agent" may include, for example, peptides, polypeptides, synthetic organic molecules, naturally-occurring organic molecules, nucleic acid molecules, and components thereof, as well as one or more detectable agent covalently or non-covalently linked to the diagnostic agent.

By a "functional fragment," as used herein in reference to polypeptide, is meant a fragment that retains at least one biological activity of the full-length polypeptide. Examples of such a biological activity are the ability to specifically bind an antigen, induce apoptosis, and/or inhibit cell proliferation. For instance, a functional fragment may specifically bind to an adenocarcinoma of the colon, a diffuse-type stomach carcinoma, an adenocarcinoma of the pancrease, and/or an adenocarcinoma of the lung, and not to a non-neoplastic cell of the same tissue type. The biological activities of a functional fragment may be determined, for example, using any one of the assays described herein.

Examples of functional fragments of an antibody are V_{L}, V_{H}, F_{V}, F_{C}, Fab, Fab', or F(ab')₂ fragments which are known to one skilled in the art (see, e.g., Huston et al., Cell Biophys. 22:189-224, 1993; and Harlow and Lane, Using Antibodies: A Laboratory Manual, Cold Spring Harbor Laboratory Press, N.Y., 1999). Desirably, a "functional fragment" has an amino acid sequence that is substantially identical to a fragment, e.g., 3, 4, 5, 10, 15, 20, 15, 30, 50, 75, or 100 contiguous amino acids, of the amino acid sequence of SEQ ID NO:1, 3, 5, or 7. In more desirable embodiments, a "functional fragment" is identical to a fragment of the sequence of SEQ ID NO:1, 3, 5, or 7. Such a "functional fragment" may contain 3, 4, 5, 6, 7, 8, 9, 10, 15, 20, 15, 30, 50, 75, or 100 contiguous amino acids of SEQ ID NO:1, 3, 5, or 7, or may be the entire amino acid sequence of SEQ ID NO:1, 3, 5, or 7. In desirable embodiments, such a fragment includes one or more of the Complement Determining Regions (CDR) of the V_{H} or the V_{L} regions of the NORM-1 or NORM-2 antibody. For example, a functional fragment may include amino acids 31-35, 50-66, and/or 99-107 of SEQ ID NO:1; amino acids 23-33, 49-55, and/or 88-99 of SEQ ID NO:3; amino acids 31-35, 50-66, and/or 99-108 of SEQ ID NO:5; or amino acids 23-36, 52-58, and/or 91-101 of SEQ ID NO:7.

By a "healthy donor," as used herein, is meant an individual, e.g., a human, in whom a malignant neoplasm has not been detected. In a desirable embodiment, a "healthy donor" is an individual in whom a neoplasm has not been detected. For example, a "healthy donor" may be a human who has never been diagnosed as having a malignant neoplasm.

By "high stringency hybridization conditions" is meant, for example, hybridization at approximately 42°C in about 50% formamide, 0.1 mg/ml sheared salmon sperm DNA, 1% SDS (Sodium Dodecyl Sulfate), 2X SSC (Sodium Citrate Buffer), 10% Dextran Sulfate, a first wash at approximately 65°C in about 2X SSC, 1% SDS, followed by a second wash at approximately 65°C in about 0.1X SSC. Alternatively, "high stringency hybridization conditions" may include hybridization at approximately 42°C in about 50% formamide, 0.1 mg/ml sheared salmon sperm DNA, 0.5% SDS, 5X SSPE, 1X Denhardt's, followed by two washes at room temperature in 2X SSC, 0.1 % SDS, and two washes at between 55-60°C in 0.2X SSC, 0.1 % SDS.

A "hybridoma," as used herein, is any cell that is artificially created by the fusion of a normal cell, such as an activated lymphocyte, with a neoplastic cell, e.g., a myeloma. The hybrid cell, which results from the fusion of at least two cells, may produce a monoclonal antibody or T cell product identical to that produced by the immunologically-competent parent. In addition, these cells, like the neoplastic parent, are immortal.

By "immortalizing," as used herein, is meant fusing a primary cell to an immortal cell, thereby obtaining a cell that retains some of the properties of the primary cell, for example, antibody production, but that can be cultured indefinitely. Exemplary primary cells that may be immortalized include cells derived from the spleen, a lymph node, the blood, or the bone marrow of a healthy donor. Desirably, the primary cell is a lymphocyte derived from the spleen or a lymph node of a healthy donor. Exemplary immortal cells myelomas and heteromyelomas. For instance, a desirable heteromyeloma for immortalizing a primary cell may be HAB-1 (Faller, et al., Br. J. Cancer 62:595-598, 1990), CB-F7 (Delvig et al., Hum. Antibodies Hybridomas 6:42-46, 1995), K6H6B5 (Delvig et al., Hum. Antibodies Hybridomas 6:42-46, 1995), H7NS.934 (Delvig et al., Hum. Antibodies Hybridomas 6:42-46, 1995), SHM-D33 (Bron et al., Proc. Natl. Acad. Sci. USA 81:3214-3217, 1984), or B6B11 (Borisova et al., Vopr. Virusol. 44:172-174, 1999).

"Inhibiting cell proliferation," as used herein, refers to a reduction in the rate of cell division of a cell in comparison with the normal rate of cell division of that type of cell under the same conditions. Inhibition of cell proliferation may be assayed using a number of methods standard in the art, for example, the MTT cell proliferation assay described herein, BrdU incorporation, and ³H thymidine uptake. Such assays are described, for example, in Ausubel et al., Current Protocols in Molecular Biology, Wiley Interscience, New York, 2001; and Sambrook et al., Molecular Cloning: A Laboratory Manual, 3rd edition, Cold Spring Harbor Laboratory Press, N.Y., 2001. Desirably, the inhibition of cell proliferation is 20%, 40%, 50%, or 75%. In desirable embodiments, the inhibition of cell proliferation is 80%, 90%, 95%, or even a complete inhibition of cell proliferation.

"Inducing apoptosis," as used herein, refers to the appearance of characteristics in a cell that are well defined in the art (see, e.g., Wyllie et al., Br. J. Cancer 80 Suppl. 1:34-37, 1999; Kerr et al., Br. J. Cancer 26:239-257, 1972). These characteristics include morphological characteristics, such as membrane blebbing, DNA condensation, as well as changes in F-actin content, mitochondrial mass, and membrane potential. The induction of apoptosis may be assayed using a number of methods standard in the art, for example, a cell death ELISA, TUNEL staining, DNA stains, e.g., Hoechst 33258, and staining with various vital dyes such as acridine orange, Mito Tracker Red^{®} staining (Molecular Probes, Eugene, OR), and Annexin V^{®} staining (Becton Dickinson, NJ). As used herein "inducing apoptosis" refers to an increase in the number of cells undergoing apoptosis when compared with a control cell population under the same conditions. For instance, the increase of apoptosis may be 10%, 20%, 40%, 50%, or 75%. In desirable embodiments, the induction of apoptosis results in an increase of apoptosis that is 2-fold, 3-fold, 10-fold, or even 100-fold over that seen in a control cell population.

A "neoplastic cell," as used herein, refers to a cell which is undergoing cell division, not undergoing apoptosis, or both, under inappropriate conditions. For example, a "neoplastic cell" may undergo cell division when a corresponding non-neoplastic cell does not undergo cell division, or, alternatively, a "neoplastic cell" may not respond to normal cell-cycle checkpoint controls.

A "proliferative disease," as used herein, refers to any disorder that results in the abnormal proliferation of a cell. Specific examples of proliferative diseases are various types of neoplasms, such as stomach adenocarcinoma, colorectal adenocarcinoma, lung adenocarcinoma, and adenocarcinoma of the pancreas. However, proliferative diseases may also be the result of the cell becoming infected with a transforming virus.

A "protein purification tag," as used herein, is a peptide, e.g., an epitope tag, that is covalently or non-covalently added to a protein to aid in the purification of the protein. Desirably such peptides bind with high affinity to an antibody or to another peptide such as biotin or avidin. Commercially available examples of epitope tags include His-tags, HA-tags, FLAG^{®}-tags, and c-Myc-tags. However, any epitope that is recognized by an antibody also may be used as a protein purification tag. See, for example, Ausubel et al., Current Protocols in Molecular Biology, Wiley Interscience, New York, 2001; and Sambrook et al., Molecular Cloning: A Laboratory Manual, 3rd edition, Cold Spring Harbor Laboratory Press, N.Y., 2001. Protein purification tags may be cleaved from a protein, for example, by using an enzyme, e.g., thrombin, or a chemical, e.g., cyanogen bromide.

By "specifically binds" and "specifically recognizes" as used herein in reference to a polypeptide, e.g., an antibody, is meant an increased affinity of a polypeptide for a particular protein, e.g., an antigen, relative to an equal amount of any other protein. For example, an antibody, e.g., the NORM-1 or NORM-2 human monoclonal antibody, that specifically binds to at least one of EPLC-272H (DSMZ Accession No. ACC 383), Colo-699 (DSMZ Accession No. ACC 196), CACO-2 (ATCC Accession No. HBT-37; DSMZ Accession No. ACC 169), Colo-206F (DSMZ Accession No. ACC 21), 23132/87 (DSMZ Accession No. ACC 201), ASPC-1 (ATCC Accession No. CRL-1682), DU-145 (DSMZ Accession No. ACC 261, ATCC Accession No. HTB-81), and BM10604 (DSMZ Accession No. ACC 298) cells desirably has an affinity for its antigen that is least 2-fold, 5-fold, 10-fold, 30-fold, or 100-fold greater than for an equal amount of any other antigen, including related antigens. Binding of a polypeptide to another polypeptide may be determined as described herein, and by any number of standard methods in the art, e.g., Western analysis, ELISA, or co-immunoprecipitation.

By "substantially identical" is meant a polypeptide or nucleic acid exhibiting at least 80%, 85%, 90%, or 95% identity to a reference amino acid (e.g., the sequence of SEQ ID NO:1, 3, 5, or 7) or nucleic acid sequence (e.g., the sequence of SEQ ID NO:2, 4, 6, or 8), or a fragment thereof. In desirable embodiments, the polypeptide or nucleic acid sequence is at least 98%, 99%, 99.4%, 99.5%, 99.6 %, 99.7%, 99.8%, 99.9%, or even 100% identical to a reference amino acid or nucleic acid sequence. For polypeptides, the length of comparison sequences will generally be at least 3, 4, 5, 6, 8, 10, or 15 amino acids and desirably at least 20 or 25 contiguous amino acids. In more desirable embodiments, the length of comparison sequences is at least 30, 50, 75, 90, or 95 contiguous amino acids, or even the full-length amino acid sequence. For nucleic acids, the length of comparison sequences will generally be at least 9, 10, 12, 15, 18, 20, 24, or 25 contiguous nucleotides, and desirably at least 30 contiguous nucleotides. In more desirable embodiments, the length of comparison sequences is at least 50, 75, 150, 225, 270, 280, 285, or 290 contiguous nucleotides, or even the full-length nucleotide sequence.

Sequence identity may be measured using sequence analysis software on the default setting (e.g., Sequence Analysis Software Package of the Genetics Computer Group, University of Wisconsin Biotechnology Center, 1710 University Avenue, Madison, WI 53705). Such software may match similar sequences by assigning degrees of homology to various substitutions, deletions, and other modifications. Conservative substitutions typically include substitutions within the following groups: glycine, alanine, valine, isoleucine, leucine; aspartic acid, glutamic acid, asparagine, glutamine; serine, threonine; lysine, arginine; and phenylalanine, tyrosine.

Multiple sequences may also be aligned using the Clustal W(1.4) program (produced by Julie D. Thompson and Toby Gibson of the European Molecular Biology Laboratory, Germany and Desmond Higgins of European Bioinformatics Institute, Cambridge, UK) by setting the pairwise alignment mode to "slow," the pairwise alignment parameters to include an open gap penalty of 10.0 and an extend gap penalty of 0.1, as well as setting the similarity matrix to "blosum." In addition, the multiple alignment parameters may include an open gap penalty of 10.0, an extend gap penalty of 0.1, as well as setting the similarity matrix to "blosum," the delay divergent to 40%, and the gap distance to 8.

By "purified" or "isolated" is meant separated from other components that naturally accompany it. Typically, a factor is "purified" or "isolated" when it is at least 50%, by weight, free from proteins, antibodies, and naturally-occurring organic molecules with which it is naturally associated, or in reference to a nucleic acid molecule, is free from the nucleic acid sequences that naturally flank the sequence of the nucleic acid molecule in the genome of an organism. Desirably, the factor is at least 75%, more desirably, at least 90%, and most desirably, at least 99%, by weight, pure. A substantially pure factor may be obtained by chemical synthesis, separation of the factor from natural sources, or production of the factor in a recombinant host cell that does not naturally produce the factor. Proteins, vesicles, and organelles may be purified by one skilled in the art using standard techniques, such as those described by Ausubel et al. (Current Protocols in Molecular Biology, Wiley Interscience, New York, 2001). The factor is desirably at least 2, 5, or 10 times as pure as the starting material, as measured using polyacrylamide gel electrophoresis, column chromatography, optical density, HPLC analysis, or Western analysis (Ausubel et al., Currant Protocols in Molecular Biology, Wiley Interscience, New York, 2001). Desirable methods of purification include immunoprecipitation, column chromatography such as immunoaffinity chromatography and nickel affinity columns, magnetic bead immunoaffinity purification, and panning with a plate-bound antibody.

By "vector" or "expression vector" is meant an expression system, a nucleic acid-based shuttle vehicle, a nucleic acid molecule adapted for nucleic acid delivery, or an autotonomous self-replicating circular DNA (e.g., a plasmid). When a vector is maintained in a host cell, the vector can either be stably replicated by the cell during mitosis as an autonomous structure, incorporated into the genome of the host cell, or maintained in the host cell's nucleus or cytoplasm.

Other features and advantages of the invention will be apparent from the following Detailed Description, the Drawings, and the Claims.

### Brief Description of the Drawings

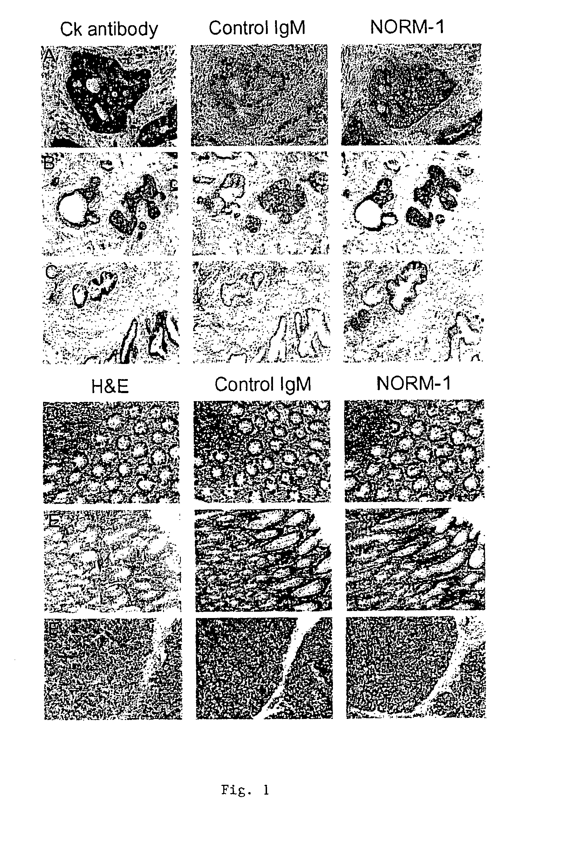

Figures 1A-1F are a series of images showing immunohistochemical staining of NORM-1 and control antibodies on different carcinoma and normal tissues. Paraffin sections were stained with hematoxylin-eosin ("H&E"), positive control antibodies (anti-cytokeratin 7 for adenocarcinoma of the pancreas and anti-cytokeratin 8 for adenocarcinoma of the stomach and colon ("Ck")), unrelated human IgM antibody as a negative control (" Control IgM"), and NORM-1. Figure 1A shows staining of an adenocarcinoma of the colon; Figure 1B shows staining of a diffuse-type stomach carcinoma; Figure 1C shows staining of an adenocarcinoma of the pancreas; Figure 1D shows staining of normal colon tissue; Figure 1E shows staining of normal gastric tissue; and Figure 1F shows staining of normal pancreatic tissue. The original magnification for these images was 100x.

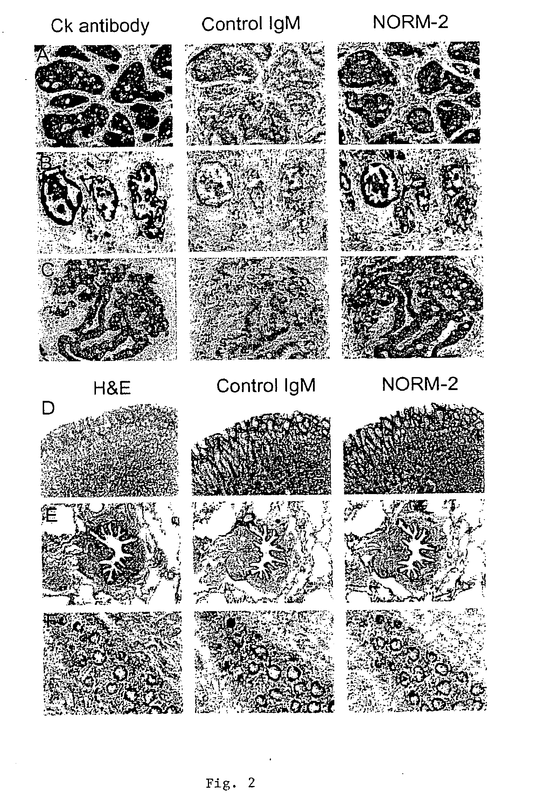

Figures 2A-2F are a series of images showing immunohistochemical staining of NORM-2 and control antibodies on different carcinoma and normal tissues. Paraffin sections were stained with hematoxylin-eosin ("H&E"), positive control antibody (anti-cytokeratin 8 ("Ck")), unrelated human IgM antibody as a negative control ("Control IgM"), and NORM-2. Figure 2A shows staining of a diffuse-type stomach carcinoma; Figure 2B shows staining of an adenocarcinoma of the lung; Figure 2C shows staining of an adenocarcinoma of the colon; Figure 2D shows staining of normal gastric tissue; Figure 2E shows staining of normal lung tissue; and Figure 2F shows staining of normal colon tissue. The original magnification for these images was 100x.
Figure 3 is a graph depicting the functional analysis of antibodies NORM-1 and NORM*-*2 *in vitro.* The consequences of antibody treatment on the proliferation of stomach carcinoma cell line 23132/87 (DSMZ Acession No. ACC 201) was measured using an MTT proliferation assay. In Control 1 complete growth medium without an antibody was added to the cells, and in Control 2 complete growth medium containing an unrelated IgM antibody at a similar concentration was added to the cells.
Figure 4 is a graph showing that the NORM-1 and NORM-2 antibodies induce apoptosis. In these experiments, apoptosis of 23132/87 cells was detected using the Cell Death Detection ELISA^{PLUS} apoptosis assay (Roche, Mannheim, Germany). In Control 1 complete growth medium without an antibody was added to the cells, and in Control 2 complete growth medium containing an unrelated IgM antibody at a similar concentration was added to the cells.
Figures 5A and 5B are a series of graphs of the results of cell death enzyme-linked immunosorbent assays (ELISA) showing that the NORM-1 monoclonal antibody induces apoptosis of 23132/87 cells after 24 hours (Fig. 5A) and 48 hours (Fig. 5B) of incubation.
Figures 6A and 6B are a series of graphs of the results of cell death ELISAs showing that the NORM-2 monoclonal antibody induces apoptosis of 23132/87 cells after 24 hours (Fig. 6A) and 48 hours (Fig. 6B) of incubation.
Figure 7 is the amino acid sequence (SEQ ID NO:1) and the nucleic acid sequence (SEQ ID NO:2) of the variable region of the heavy chain of human monoclonal antibody NORM-1. The D-region and the J-region are indicated and Complement Determining Regions ("CDR") 1 to 3 are also shown.
Figure 8 is the amino acid sequence (SEQ ID NO:3) and the nucleic acid sequence (SEQ ID NO:4) of the variable region of the light chain of human monoclonal antibody NORM-1. The J-region is indicated and CDR1 to 3 are also shown.
Figure 9 is the amino acid sequence (SEQ ID NO:5) and the nucleic acid sequence (SEQ ID NO:6) of the variable region of the heavy chain of human monoclonal antibody NORM-2. The D-region and J-region are indicated and CDR1 to 3 are also shown.
Figure 10 is the amino acid sequence (SEQ ID NO:7) and the nucleic acid sequence (SEQ ID NO:8) of the variable region of the light chain of human monoclonal antibody NORM-2. The J-regions is indicated and CDR1 to 3 are also shown.

### Detailed Description

The present invention features polypeptides, such as antibodies, identified using cells from healthy donors, and their use in the treatment and diagnosis of neoplasms. In addition, the invention features methods of identifying neoplasm-specific polypeptides using cells obtained from healthy donors. We have characterized two human monoclonal antibodies (NORM-1 and NORM-2), that we obtained using the methods of the present invention, and that specifically recognize a number of carcinomas. Not only do these monoclonal antibodies recognize these neoplasms, but, upon binding to a cell, they can induce apoptosis of neoplastic cells, inhibit their proliferation, or even both.

Thus, the NORM-1 and NORM-2 monoclonal antibodies, and other antibodies, or fragments thereof, that are specific for the antigen recognized by these antibodies, may be used in a variety of methods for diagnosing and treating a neoplasm.

The cell lines that produce the human NORM-1 (Accession No. DSM ACC2624) and NORM-2 (Accession No. DSM ACC2626) monoclonal antibodies were deposited on November 6, 2003 at the German Collection of Microorganisms and Cell Cultures ("DSMZ" - Deutsche Sammlung von Mikroorganismen und Zellkulturen GmbH, Mascheroder Weg 1b, 38124 Braunschweig, Germany) under the terms of the Budapest Treaty.

### Antibodies and Polypeptides

Antibodies play an essential role in maintaining the health of an individual. In particular, antibodies are present in serum and bind to and help eliminate diverse pathogens such as bacteria, viruses, and toxins. Antibodies consist of Y-shaped protein structures built from two heavy chains and two light chains. Each chain has a modular construction: each light chain consists of two domains, and each heavy chain has at least four domains. The antigen binding site is fashioned by one domain from the heavy chain (V_{H} domain) and one domain from the light chain (V_{L} domain). Indeed, small antigen binding fragments can be prepared by linking these two domains, either associated non-covalently, or covalently via disulphide bonds or a peptide linker. The antigen binding domains are more variable in amino acid sequence than the other domains of the antibody, and are therefore termed variable (V) domains, in contrast to the constant (C) domains. The constant domains of the antibody are responsible for triggering antibody effector mechanisms, such as complement lysis and cell-mediated killing.

Antibodies are made by B-lymphocytes in a process involving gene rearrangement. During the development of these cells, the genes encoding the variable domains are assembled from genetic elements.

In the case of the V_{H} domains there are three elements, the un-rearranged V_{H} gene, D segment, and J_{H} segment. In the case of the V_{L} domains, there are two elements, the un-rearranged V_{L} (V Lambda or V Kappa) gene and the J_{L} (J Lambda or J Kappa) segment. Random combination of these gene segments and random combination of the rearranged V_{H} and V_{L} domains generate a large repertoire of antibodies, capable of binding to a large diversity of equally diverse antigens. Further, the V_{H} and V_{L} regions each have three Complement Determining Regions (CDR) and four framework regions (FR). The FRs are the backbone of the antibody and the CDRs are the parts of the antibody that bind the antigen. One skilled in the art can determine the FR and CDR regions of an antibody by comparing the amino acid sequence of a number of antibodies raised in the same species (see, e.g., Altschul et al., Nucleic Acids Res. 25:3389-3402, 1997; and Kabat et al., Sequences of Proteins of Immunological Interest, 5th edition, NIH Publication No. 91-3242, U.S. Department of Health and Human Services, 1991).

In general, a polypeptide identified by the methods of the invention is an agent that binds to any one of EPLC-272H, Colo-699, CACO-2, Colo-206F, 23132/87, ASPC-1, DU-145, and BM10604 cells, but does not bind to non-neoplastic cells. The polypeptide may be an antibody, such as a human monoclonal antibody (e.g., NORM-1 or NORM-2), or a functional fragment thereof. Overall, the methods of the invention can be used to identify polypeptides that exclusively bind to both neoplastic tissues and neoplastic cells, but not to non-neoplastic tissue or cells. A polypeptide identified using the methods of the invention also may induce apoptosis of a neoplastic cell to which it binds, but not in a non-neoplastic cell, or, alternatively, the polypeptide may inhibit proliferation of the neoplastic cell it binds to, but not in a non-neoplastic cell.

Desirably, a polypeptide identified using the methods of the invention can simultaneously induce apoptosis and inhibit proliferation of neoplastic cells, but not of non-neoplastic cells. Such a polypeptide is, therefore, useful for the detection, monitoring, prevention, and treatment of cancers in mammals. Exemplary cancers amenable to treatment or diagnosis using polypeptides identified with the methods of the invention include colorectal cancer, ovarian carcinoma, squamous cell lung carcinoma, small cell lung carcinoma, lobular and ductal mammary carcinomas, melanoma, breast cancer, lung cancer, such as lung adenocarcinomas, gastric cancer, pancreatic cancer, such as pancreatic adenocarcinomas, gliomas, sarcomas, gastrointestinal cancer, brain tumors, esophageal cancer, such as esophagial squamous cell carcinomas, stomach cancer, osteosarcoma, fibrosarcomas, urinary bladder cancer, prostate cancer, such as prostate adenocarcinomas, renal cancer, ovarian cancer, testicular cancer, endometrial cancer, cervical cancer, uterine adenocarcinomas, Hodgkin's disease, lymphomas, and leukemias. Polypeptides identified using the methods of the invention are particularly useful for the detection and treatment of a stomach adenocarcinoma, colorectal adenocarcinoma, lung adenocarcinoma, and adenocarcinoma of the pancreas.

### Identification Neoplasm-Specific Polypeptides

In general, a hybridoma expressing a polypeptide that specifically binds to a neoplastic cell, but does not bind to a non-neoplastic cell (e.g., a NORM-1 or NORM-2 monoclonal antibody), may be generated by fusing lymphocytes obtained from the spleen, lymph nodes, blood, or bone marrow of a healthy donor with a myeloma or heteromyeloma cell line. Typically, lymphocytes are obtained from portions of a lymph node or spleen that was surgically removed from a healthy donor. For instance, a portion of the spleen may be removed from a healthy donor due to an accident that resulted in a splenic rupture. However, lymphocytes may also be obtained from a healthy donor's blood or bone marrow. For example, lymphocytes may be isolated from a healthy donor's blood by means of density gradient centrifugation. Thus, the methods of the invention allow for the generation of neoplasm-specific human monoclonal antibodies from lymphocytes of healthy donors without obtaining cells or tissue from a cancer patient.

In particular, lymphocytes may be prepared as cell suspensions by mechanical means and subsequently fused at, for example, a 1:2 or 1:3 ratio with a myeloma or heteromyeloma cell line under conditions that result in cell fusion. For instance, the heteromyeloma cell line HAB-1 (Faller, et al., Br. J. Cancer 62:595-598, 1990), which is generated by the fusion of a human lymphocyte with the mouse myeloma NS-0, may be used for this purpose. Other exemplary heteromyeloma cell lines include, for example, CB-F7 (Delvig et al., Hum. Antibodies Hybridomas 6:42-46, 1995), K6H6B5 (Delvig et al., Hum. Antibodies Hybridomas 6:42-46, 1995), H7NS.934 (Delvig et al., Hum. Antibodies Hybridomas 6:42-46, 1995), SHM-D33 (Bron et al., Proc. Natl. Acad. Sci. USA 81:3214-3217, 1984), and B6B11 (Borisova et al., Vopr. Virusol. 44:172-174, 1999). Following the fusion of the lymphocytes derived from the healthy donor with the heteromyeloma cell line, an antibody producing hybridoma or trioma is generated.

Cell fusion may be achieved by any method known in the art, such as, for example, the use of 40% polyethylene glycol (e.g., PEG 1500). Hybridomas may be cultured in media containing HAT (hypoxanthine-aminopterin-thymidine) and after four weeks, supernatants may be screened for antibody production using an ELISA assay. Positive clones may then be tested in attachment inhibition and binding assays using commercially available tumor cell lines. Positive clones further may be tested using immunoperoxidase staining of neoplastic and normal tissues. Thus, clones may be selected on the basis of their reactivity with neoplastic cells and not with normal cells. The antibody may be purified from mass cultures with use of ion-exchange, hydrophobic interaction, size exclusion, or affinity chromatography, as well as a combination of these methods, as described, for example, by Vollmers et al. (Oncology Reports 5:35-40, 1998). Following the production of antibodies, additional functional and immunohistochemical tests of the antibodies produced by the trioma may be performed. For example, the antibodies produced by the trioma can be tested for their ability to induce apoptosis, inhibit cellular proliferation, or both, relative to untreated control cells. The antibodies can also be tested for their ability to specifically bind the neoplastic cell lines EPLC-272H, Colo-699, CACO-2, Colo-206F, 23132/87, ASPC-1, DU-145, BM1604, relative to non-neoplastic cells.

### Production of Neoplasm-Specific Polypeptides

Once constructed, hybridomas are generally stable in growth and antibody production in standard and mass cultures (flasks, miniPerm, fermenters, etc.) for several months. Thus, hybridomas expressing polypeptides identified according to the methods of the invention can be used in any method known in the art for small scale, large scale, or commercial production of polypeptides. Levels of antibody production typically range between 0.01-0.1 mg/mL in flasks and between 0.1-0.5 mg/mL in miniPerm.

In addition, once a polypeptide has been identified using the methods of the invention, the polypeptide, e.g., an antibody, or a fragment thereof, may also be produced by expression in a host cell such as *E. coli* or yeast, e.g., S. *cerevisiae,* or a mammalian cell line. Functional fragments of polypeptides may also be generated, for example, by direct synthesis using recombinant methods. These methods are standard in the art. For example, a nucleic acid sequence may be amplified using the polymerase chain reaction (PCR). The PCR technique is known in the art and is described, for example in U.S. Patent No. 4,683,195. Using standard methods, and as described herein, the sequence of a monoclonal antibody expressed by a hybridoma or trioma may be obtained and functional fragments of the antibody may be amplified. For example, whole RNA may be isolated from a hybridoma expressing a tumor-specific monoclonal antibody. cDNA may then be generated from the RNA using reverse transcriptase and the cDNAs which contain the functional fragments of the variable regions of the heavy and light chains may be amplified using PCR. The PCR products may then be purified and cloned into expression vectors, e.g., plasmid or viral vectors. Many standard vectors are available and the selection of the appropriate vector will depend on, for example, the size of the DNA inserted into the vector and the host cell to be transfected with the vector.

The nucleic acid molecules identified using the methods of the invention may be expressed in a variety of standard vectors and host cells. Any promoter that is active in the host cell may be used to express a nucleic acid molecule. Nonetheless, for expression of an antibody or a fragment of an antibody in a mammalian cell, use of an immunoglobulin gene promoter is desirable. Methods of introducing a vector into a host cell are standard in the art and include, electroporation, use of synthetic lipid polymers, e.g., Lipofectin™, use of calcium chloride, and use of DEAE Dextran. Such methods are also described in, for example, Ausubel et al., Current Protocols in Molecular Biology, Wiley Interscience, New York, 2001; and Sambrook et al., Molecular Cloning: A Laboratory Manual, 3rd edition, Cold Spring Harbor Laboratory Press, N.Y., 2001.

### Isolation of Amino Acid Variants of a Polypeptide

Amino acid sequence variants of a polypeptide identified using the methods of the invention, such as an antibody, e.g., a NORM-1 or NORM-2 antibody, can be prepared by introducing appropriate nucleotide changes into the DNA encoding the antibody, or by *in vitro* synthesis of the desired polypeptide. Such variants include, for example, deletion, insertion, or substitution of, residues within the amino acid sequence of the NORM-1 or NORM-2 antibody. Any combination of deletion, insertion, and substitution can be made to arrive at the final construct, provided that the final construct possesses the desired characteristics, e.g., the ability to induce apoptosis of a neoplastic cell, but not a non-neoplastic cell, or the ability to inhibit the proliferation of a neoplastic cell, but not a non-neoplastic cell. The amino acid changes also may alter post-translational processes of an antibody, such as changing the number or position of glycosylation sites, altering the membrane anchoring characteristics, or modifying its susceptibility to proteolytic cleavage.

In designing amino acid sequence variants of a polypeptide, such as an antibody, the location of the mutation site and the nature of the mutation will depend on characteristic(s) to be modified. The sites for mutation can be modified individually or in series, e.g., by substituting first with conservative amino acid choices and then with more radical selections depending upon the results achieved, or deleting the target residue.

A useful method for identification of specific residues or regions for mutagenesis in a polypeptide is called "alanine scanning mutagenesis" and is described, for example, by Cunningham and Wells (Science 244:1081-1085, 1989). Here, a residue or group of target residues are identified (e.g., charged residues such as arg, asp, his, lys, and glu) and replaced by a neutral or negatively charged amino acid (most desirably alanine or polyalanine) to affect the interaction of the amino acids with the surrounding aqueous environment in or outside the cell. The domains demonstrating functional sensitivity to the substitutions then are refined by introducing further or other variants at or for the sites of substitution. Thus, while the site for introducing an amino acid sequence variation is predetermined, the nature of the mutation need not be predetermined. For instance, to optimize the performance of a mutation at a given site, alanine scanning or random mutagenesis may be conducted at the target codon or region and the expressed variants are screened for, e.g., the ability to induce apoptosis of a neoplastic cell and not a non-neoplastic cell, or to inhibit the proliferation of a neoplastic cell and not a non-neoplastic cell.

The sites of greatest interest for substitutional mutagenesis include sites identified as affecting the biological activity of a polypeptide. These sites, especially those falling within a sequence of at least three other identically conserved sites, may be substituted in a relatively conservative manner. For instance, ala may be substituted with val, leu, or ile; arg may be substituted with lys, gln, or asn; asn may be substituted with gln, his, lys, or arg; asp may be substituted with glu; cys may be substituted with ser; gln may be substituted with asn; glu may be substituted with asp; gly may be substituted with pro; his may be substituted with asn, gln, lys, or arg; ile may be substituted with leu, val, met, ala, or phe; leu may be substituted with ile, val, met, ala, or phe; lys may be substituted with arg, gln, or asn; met may be substituted with leu, phe, or ile; phe may be substituted with leu, val, ile, or ala; pro may be substituted with gly; ser may be substituted with thr; thr may be substituted with ser; trp may be substituted with tyr; tyr may be substituted with trp, phe, thr, or ser; and val may be substituted with ile, leu, met, or phe.

### Conjugation of the Antibody with a Detectable Agent

If desired, a polypeptide identified using the methods of the invention such as an antibody (e.g., monoclonal antibody, such as NORM-1 or NORM-2), or a fragment thereof, may be linked to a detectable agent to facilitate the purification of the polypeptide as well as the diagnosis, monitoring, or treatment of a neoplasm in a mammal in need thereof. The selection of suitable detectable agent will depend on the intended use of the polypeptide and will be apparent to those of ordinary skill in the art. Detectable agents according to the invention include, for example, protein purification tags, cytotoxins, enzymes, paramagnetic labels, enzyme substrates, co-factors, enzyme inhibitors, dyes, radionuclides, chemiluminescent labels, fluorescent markers, growth inhibitors, and biotin.

A protein purification tag may be conjugated to a polypeptide identified using the methods of the invention, to facilitate isolation of the polypeptide. Examples of tags that can be used include His-tags, HA-tags, FLAG^{®}-tags, and c-Myc tags. An enzymatic or a chemical cleavage site may be engineered between the polypeptide and the tag moiety so that the tag can be removed following purification. Suitable toxins include diphtheria toxin, Pseudomonas exotoxin A, ricin, and cholera toxin. Examples of suitable enzyme labels include malate hydrogenase, staphylococcal nuclease, delta-5-steroid isomerase, alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholinesterase. Examples of suitable radioisotopic labels include ³H, ¹²⁵I, ¹³¹I , ³²p , ³⁵S and ¹⁴C. Desirably, the radioisotope will emit in the 10-5,000 kev range, more desirably 100-500 kev. Paramagnetic isotopes may also be conjugated to the polypeptide and *used in vivo* for the diagnosis and treatment of cancer. The use of such conjugated antibodies may be for *in vivo* nuclear magnetic resonance imaging. Such methods are known in the art (see, for example, Schaefer et al., JACC 14:472-480, 1989; Shreve et al., Magn. Reson. Med. 3:336-340, 1986; Wolf, Physiol. Chem. Phys. Med. NMR 16:93-95, 1984; Wesbey et al., Physiol. Chem. Phys. Med. NMR 16:145-155, 1984; and Runge et al., Invest. Radiol. 19:408-415, 1984). Alternatively, the radiolabeled antibody may also be used in radioimmunoguided surgery (RIGS), which involves the surgical removal of any tissue the labeled antibody binds to. Thus, the labeled antibody guides the surgeon towards neoplastic tissue by distinguishing it from non-neoplastic tissue. Radiolabels useful for tumor imaging are preferably short-lived radioisotopes. Various radioactive metals with half-lives ranging from 1 hour to 11.4 days are available for conjugation to antibodies, such as scandium-47 (3.4 days), gallium-67 (2.8 days), gallium-68 (68 minutes), technetium-99m (6 hours), indium-111 (3.2 days), and radium-223 (11.4 days), of which gallium-67, technetium-99m, and indium-111 are preferable for gamma camera imaging, gallium-68 is preferable for positron emission tomography, and scandium-47 and radium-223 (and other alpha-emitting radionuclides) are preferable for tumor therapy.

Examples of suitable fluorescent markers include fluorescein, isothiocyalate, rhodamine, phycoerythrin, phycocyanin, allophycocyanin, ophthaldehyde, and fluorescamine. Examples of chemiluminescent markers include a luminal label, isoluminal label, aromatic acridinium ester label, imidazole label, acridinium salt label, oxalate ester label, luciferin label, luciferase label, and aequorin label. Those of ordinary skill in the art would know of other suitable labels, which may be employed in accordance with the present invention. Conjugation of these detectable agents to a polypeptide identified using the methods of the invention, such as monoclonal antibodies, or fragments thereof, can be accomplished using standard techniques known in the art. Typical antibody conjugation techniques are described by Kennedy et al. (Clin. Chim. Acta 70, 1-31, 1976) and Schurs et al. (Clin. Chim. Acta 81, 1-40 , 1977) and include, for example, the glutaraldehyde method, the periodate method, the dimaleimide method, the m-maleimidobenzyl-N-hydroxy-succinimide ester method. Antibodies may be radiolabeled by any of several techniques known to the art, described, for example, in U.S. patent No. 4,444,744. All of these methods are incorporated by reference herein.

In all methods of treatment of the present invention, it is understood that mixtures of different or the same labeled polypeptides specific to different antigens or different epitopes of the same antigen associated with the same or different tumor or tumor cell types may be used. Such a combination may enhance detection, localization, and/or therapy in certain cases, and can also increase the range of a broad screen for more than one neoplasm or type of neoplasm.

### Polypeptides Conjugated to Anti-Tumor Agents

Although a polypeptide identified using the methods of the invention may induce apoptosis of neoplastic cells, inhibit cellular proliferation of neoplastic cells, or both, the polypeptide may in addition be conjugated to an agent that kills neoplastic cells or that inhibits their proliferation. The targeting ability of the polypeptide, such as an antibody or fragment thereof, results in the delivery of the cytotoxic or anti-proliferative agent to the tumor to enhance the destruction of the tumor. The polypeptide therefore may be used for the treatment and prevention of a neoplasm in a mammal, such as a human patient. The cytotoxic agent linked to the polypeptide may be any agent that destroys or damages a tumor cell or tumor to which the polypeptide has bound. Examples of such agents include chemotherapeutic agents or radioisotopes, enzymes which activate a pro-drug, or a cytokine.

Suitable chemotherapeutic agents are known to those skilled in the art and include, for example, taxol, mithramycin, deoxyco-formycin, mitomycin-C, L-asparaginase, interferons (especially IFN-alpha), etoposide, teniposide, anthracyclines (e.g., daunomycin and doxorubicin), methotrexate, vindesine, neocarzinostatin, cis-platinum, chlorambucil, cytosine arabinoside, 5-fluorouridine, melphalan, ricin, and calicheamicin. The chemotherapeutic agents may be conjugated to the antibody using conventional methods known in the art.

Suitable radioisotopes for use as cytotoxic agents are also known to those skilled in the art and include, for example, ¹³¹I, or an astatine such as ²¹¹At. These isotopes may be attached to the polypeptide, either covalently or non-covalently, using conventional techniques known in the art.

Alternatively, the cytotoxic agent may also be an enzyme, which activates a pro-drug. This allows the conversion of an inactive pro-drug to its active, cytotoxic form at the tumor site and is called "antibody-directed enzyme pro-drug therapy" (ADEPT). Thus, the polypeptide-enzyme conjugate may be administered to the patient and allowed to localize in the region of the tumor to be treated. The pro-drug is then administered to the patient such that conversion to the cytotoxic drug is localized in the region of the tumor to be treated under the influence of the localized enzyme. An exemplary enzyme is bacterial carboxypeptidase G2 (CPG2) the use of which is described in, for example, WO 88/07378. The polypeptide-enzyme conjugate may, if desired, be modified in accordance with the teaching of WO 89/00427, such as to accelerate its clearance from areas of the body that are not in the vicinity of a neoplasm. The polypeptide-enzyme conjugate may also be used in accordance with WO 89/00427, for example, by providing an additional component, which inactivates the enzyme in areas of the body that are not in the vicinity of the tumor.

As another alternative, the cytotoxic agent conjugated to a polypeptide identified using the methods of the invention may also be a cytokine such as interleukin-2 (IL-2), interleukin-4 (IL-4), or tumor necrosis factor alpha (TNF-alpha). The polypeptide targets the cytokine to the tumor so that the cytokine mediates damage to or destruction of the tumor without affecting other tissues. The cytokine may be fused to the polypeptide at the DNA level using conventional recombinant DNA techniques.

In addition, any inhibitor of cell proliferation. e.g., genistein, tamoxifen, or cyclophosphamide, may be conjugated with a polypeptide identified using the methods of the invention.

### Dosage

With respect to the therapeutic methods of the invention, it is not intended that the administration of a polypeptide of the invention to a patient be limited to a particular mode of administration, dosage, or frequency of dosing; the present invention contemplates all modes of administration, including intramuscular, intravenous, intraperitoneal, intravesicular, intraarticular, intralesional, subcutaneous, or any other route sufficient to provide a dose adequate to decrease the number of neoplastic cells by inducing apoptosis of neoplastic cells, by inhibiting proliferation of tumor cells, or both. The compound(s) may be administered to the patient in a single dose or in multiple doses. When multiple doses are administered, the doses may be separated from one another by, for example, one day, two days, one week, two weeks, or one month. For example, the polypeptide (e.g., a monoclonal antibody, such as NORM-1 or NORM-2) may be administered once a week for, e.g., 2, 3, 4, 5, 6, 7, 8, 10, 15, 20, or more weeks. It is to be understood that, for any particular subject, specific dosage regimes should be adjusted over time according to the individual need and the professional judgment of the person administering or supervising the administration of the compositions. The precise dose will vary dependent on the polypeptide used, the density, on the tumor surface, of the ligand to which the polypeptide binds, and the rate of clearance of the polypeptide. For example, the dosage of the NORM-1 or NORM-2 antibody can be increased if the lower dose does not provide sufficient anti-neoplastic activity. Conversely, the dosage of the NORM-1 or NORM-2 antibody can be decreased if the neoplasm is cleared from the patient.

While the attending physician ultimately will decide the appropriate amount and dosage regimen, a therapeutically effective amount of a polypeptide, such as a monoclonal antibody or a fragment thereof, may be, for example, in the range of about 0.1 mg to 50 mg/kg body weight/day or 0.70 mg to 350 mg/kg body weight/week. Desirably a therapeutically effective amount is in the range of about 0.50 mg to 20.0 mg/kg, and more desirably in the range of about 0.50 mg to 15.0 mg/kg, for example, about 0.2, 0.3, 0.5, 1.0, 1.5, 2.0, 2.5, 3.0, 3.5, 4.0, 4.5, 5.0, 5.5, 6.0, 7.0, 8.0, 8.5, 9.0, 10.0, 11.0, 12.0, 13.0, 14.0, or 15.0 mg/kg body weight administered daily, every other day, or twice a week.

For instance, a suitable dose is an amount of the polypeptide that, when administered as described above, is capable of inducing apoptosis, and is at least 20% above the basal (i.e., untreated) level. In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. According to this invention, the administration of the polypeptide can induce neoplastic cell apoptosis by at least 20%, 40%, 50%, or 75% above that of an untreated control as measured by any standard assay known in the art. More desirably, apoptosis is induced by 80%, 90%, 95%, or even 100% above that of an untreated control. Alternatively, the administration of the polypeptide can inhibit neoplastic cell proliferation by at least 20%, 40%, 50%, or 75% below that of an untreated control as measured by any standard assay known in the art. More desirably, proliferation is inhibited by 80%, 90%, 95%, or even 100% below that of an untreated control. Most desirably, the polypeptide can simultaneously inhibit proliferation and induce apoptosis of neoplastic cells relative to untreated control cells.
Such responses can be monitored by any standard technique known in the art, including those described herein. In general, for pharmaceutical compositions, the amount of antibody present in a dose ranges from about 25 µg to 5 mg per kg of host. Suitable dose sizes will vary with the size of the patient, but will typically range from about 0.1 mL to about 5 mL.

### Formulation of Pharmaceutical Compositions

A polypeptide identified using the methods of the invention may be administered by any suitable means that results in a concentration having anti-neoplastic properties upon reaching the target region. The polypeptide may be contained in any appropriate amount in any suitable carrier substance, and is generally present in an amount of 1-95% by weight of the total weight of the composition. The composition may be provided in a dosage form that is suitable for parenteral (e.g., subcutaneous, intravenous, intramuscular, or intraperitoneal) administration route. The pharmaceutical compositions may be formulated according to conventional pharmaceutical practice (see, e.g., Remington: The Science and Practice of Pharmacy (20th ed.), ed. A.R. Gennaro, Lippincott, Williams & Wilkins, 2000 and Encyclopedia of Pharmaceutical Technology, eds. J. Swarbrick and J. C. Boylan, 1988-1999, Marcel Dekker, New York).

The pharmaceutical composition may be administered parenterally by injection, infusion or implantation (subcutaneous, intravenous, intramuscular, intraperitoneal, or the like) in dosage forms, formulations, or via suitable delivery devices or implants containing conventional, non-toxic pharmaceutically acceptable carriers and adjuvants. If the neoplastic cells are in direct contact with the blood (e.g., leukemias), or if the tumor is only accessible by the bloodstream then the intravenous (I.V.) route may be used. In cases in which tumors grow in confined spaces such as the pleural cavity or the peritoneal cavity, the polypeptide may be directly administered into the cavity rather than into the blood stream. The formulation and preparation of such compositions are well known to those skilled in the art of pharmaceutical formulation. Formulations can be found, for example, in Remington (The Science and Practice of Pharmacy (20th ed.), ed. A.R. Gennaro, Lippincott, Williams & Wilkins, 2000 and Encyclopedia of Pharmaceutical Technology, eds. J. Swarbrick and J. C. Boylan, 1988-1999, Marcel Dekker, New York).

### Diagnosis and Monitoring Cancer Progression

As discussed above, aspects of the present invention are directed to methods of detecting or diagnosing a neoplasm in a mammal, preferably a human patient. Typically, any neoplasm in which administration of a polypeptide identified using the screening methods of the invention causes an induction in apoptosis or a reduction in proliferation are amenable to the methods of diagnosis described herein.

Polypeptides identified using the screening methods of the invention are particularly useful because they specifically bind neoplasms or neoplastic cells, and not normal cells or tissue. Accordingly, such polypeptides can bind to neoplastic cells within the tumor, but not the normal surrounding tissue, thus allowing the detection, the treatment, or both, of a neoplasm in a mammal. For instance, one may use a polypeptide identified using the methods of the invention to determine if a biopsy removed the entire tumor by verifying that no cells bound by the polypeptide remain in the patient or, by verifying that tumor removed from the patient is entirely surrounded by cells that are not bound by the polypeptide.

It is understood that to improve the sensitivity of detection, multiple neoplastic markers may be assayed within a given sample or individual. Thus, polypeptides such as antibodies or functional fragments specific for different antigens may be combined within a single assay, or in multiple assays.
Further, multiple primers or probes specific to neoplasms may be used concurrently. The selection of markers may be based on routine experiments to determine combinations that results in optimal sensitivity.

### In Vitro Detection of a Neoplasm

In general, the diagnosis of a neoplasm in a mammal involves obtaining a biological sample from the mammal (e.g., human patient), contacting such sample with a polypeptide identified using the methods of the invention (e.g., a monoclonal antibody, such as NORM-1 or NORM-2), detecting, in the test sample, the level of reactivity or binding of the polypeptide to neoplastic cells relative to a control sample, which corresponds to non-neoplastic cells derived from healthy tissue from the mammal in which the cancer is being diagnosed or from another patient known not to have a neoplasm. Thus, the methods of the invention are particularly useful for the detection of early stage tumors or metastases, which are otherwise undetectable. Accordingly, in addition to diagnosing a neoplasm in a patient, the methods of this invention may also be used to monitor progression of a neoplasm in a mammal. The polypeptides described herein therefore may be used as markers for the progression of a neoplasm. For this purpose, the assays described below, which are used for the diagnosis of a neoplasm, may be performed over time, and the change in the level of reactive polypeptide(s) evaluated. For example, the assays may be performed every 24-72 hours for a period of 6 months to 1 year, and thereafter performed as needed. In general, a neoplasm is progressing in those patients in whom the level of bound polypeptide detected increases over time. In contrast, the neoplasm is not progressing when the level of bound polypeptide either remains constant or decreases with time.
Alternatively, as is noted above, polypeptides identified using the methods of the invention may also be used to determine the presence of tumor cells in the mammal following tumor resection by surgical intervention to determine whether the tumor has been completely removed from the mammal.

Desirably, the polypeptide is linked to a detectable agent, which facilitates detection, or measurement of polypeptide reactivity. The biological sample is any biological material, which may contain neoplastic cells and includes, for example, blood, saliva, tissue, serum, mucus, sputum, urine, or tears. The biological sample may also be a tissue section, which may be fixed tissue, fresh tissue, or frozen tissue. A neoplasm is detected or diagnosed in the mammal from which the sample was obtained if there is an increase in the level of reactivity of the antibody with the biological sample over the control sample. Such increase is at least 10%, 20%, 30%, 40%, 50%, or more than 50% over control levels. The level of binding or reactivity can be determined by any method known in the art and is described in further detail below.

### In Vitro Diagnostic Assays

The diagnosis of neoplasms using a polypeptide of the invention may be performed by any method known to those of ordinary skill in the art for using a binding agent to detect polypeptide markers in a sample. See, e.g., Harlow and Lane, Using Antibodies: A Laboratory Manual, Cold Spring Harbor Laboratory Press, N.Y., 1999. For example, the polypeptide may be used for enzyme-linked immunosorbent assay (ELISA), Western blotting, or *in situ* detection of tumor cells in a tissue sample. For instance, the ELISA assay typically involves the use of a polypeptide, such as an antibody, immobilized on a solid support to bind to the tumor cells in the biological sample. The bound tumor cell may then be detected using a detection reagent that contains a reporter group and that specifically binds to the antibody/tumor cell complex.
Such detection reagents include, for example, any binding agent that specifically binds to the antibody, such as an anti-immunoglobulin, protein G, protein A, or a lectin. Alternatively, a competitive assay may be utilized, in which the polypeptide is an antibody and in which the antigens, to which the antibody is specific to is labeled with a reporter group and allowed to bind to the immobilized antibody after incubation of the antibody with the biological sample. The extent to which components of the sample inhibit the binding of the labeled antigens to the antibody is indicative of the reactivity of the sample with the immobilized antibody. Diagnosis of a neoplasm in a patient may also be determined by a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group. For example, to determine the presence or absence of a neoplasm, such as a stomach adenocarcinoma, the signal detected from the reporter group that remains bound to the solid support is generally compared to a signal that corresponds to a predetermined cut-off value. The cut-off value for the detection of a neoplasm is the average mean signal obtained when the antibody is incubated with samples from patients without a neoplasm.

The method employed for detecting the reporter group depends upon the nature of the reporter group. For radioactive groups, scintillation counting or autoradiographic methods may be used. Spectroscopic methods may be used to detect dyes, luminescent groups and fluorescent groups.
Biotin may be detected using avidin, coupled to a different reporter group (commonly a radioactive or fluorescent group or an enzyme). Enzyme reporter groups may generally be detected by the addition of substrate (generally for a defined period of time), followed by spectroscopic or other analysis of the reaction products.

Polypeptides identified using the methods of the invention may also be employed histologically for *in situ* detection or quantitative determination of tumor cells, for example, by immunofluorescence or immunoelectron microscopy. *In situ* detection or determination may be accomplished by removing a tissue specimen from a patient and allowing a labeled antibody to bind to any tumor cell in the specimen. Using such a procedure not only allows the detection of neoplastic cells in a sample, but also allows for the determination of their spatial distribution. As another example, the biological sample can be a smear of biological material containing neoplastic cells on a slide, and the detection of neoplastic cells in the biological material is achieved by examining the smear with a microscope or by fluocytometry.

### In Vivo detection of a Neoplasm

Alternatively, the antibody of the invention may also be used *in vivo* for detecting and localizing a neoplasm. Such a method may involve injecting a mammal, desirably a human subject, parenterally with a polypeptide identified using the methods of the invention, such as NORM-1 or NORM-2, which has been labeled with a detectable agent, and is described, for instance, in U.S. Patent No. 4,444,744. For example, the polypeptide can be radiolabeled with a pharmacologically inert radioisotope and administered to the patient. The activity of the radioisotope can be detected in the mammal using a photoscanning device, and an increase in activity relative to a control reflects the detection and localization of a neoplasm.

### Treatment

In addition to the diagnosis and monitoring of neoplasms in mammals, the present invention also features methods for treating neoplasms in a mammal, desirably a human patient. The method generally involves the administration of a biologically effective amount of a polypeptide identified using the methods of the invention to the patient. The polypeptide is typically administered to the mammal by means of injection using any routes of administration such as by intrathecal, subcutaneous, submucosal, or intracavitary injection as well as for intravenous or intraarterial injection. Thus, the polypeptide may be injected systemically, for example, by the intravenous injection of the polypeptide such as the NORM-1 or NORM-2 antibody into the patient's bloodstream or alternatively, the polypeptide can be directly injected at the site of the neoplasm or at a location in proximity to the neoplastic cells.

In general, and as discussed above, binding of a polypeptide identified using the methods of the invention to neoplastic cells results in an induction in apoptosis, a reduction in cellular proliferation, or both relative to the control sample. Alternatively, the antibodies may also activate the complement pathway, which ultimately causes holes to be punctured into the cellular membrane, resulting in cell death.

If desired, the polypeptides may also be conjugated to drugs or toxins as described above. Once attached to the cell surface, the conjugate may be engulfed into the cell cytoplasm where cell enzymes cleave, and, thus, activate or free the drugs or toxins from the conjugate. Once released, the drugs or toxins damage the cell and irreversibly induce cell death. With respect to radiolabeled antibodies, binding to neoplastic cells and the resulting emission of radiation, at a short distance from the cell DNA, produces damage to the latter thus inducing cell death in the next replication round.
For example, after a neoplasm has been detected and localized in a subject, a higher dose of labeled antibody, generally from 25 to 250 mCi for ¹³¹I, and preferably from 50 nCi to 150 mCi per dose, based on a 70 kg patient weight, is injected. Injection may be intravenous, intraarterial, intralymphatic, intrathecal, or intracavitary, and may be repeated more than once. It may be advantageous for some therapies to administer multiple, divided doses of radiolabeled polypeptides or polypeptide mixtures, e.g., in the range of 20-120 mCi (70 kg patient), thus providing higher cell-killing doses to the neoplasm usually without effecting a proportional increase in radiation of normal tissues

Therapy using labeled polypeptides is advantageously used as a primary therapeutic treatment, but may also be used in combination with other anti-neoplastic therapies, e.g., radiation and chemotherapy, and as an adjunct to surgery. The administration of such conjugated polypeptides is particularly useful in the case where small metastases cannot be surgically removed.

### Combination of a Polypeptide with other Anti-Neoplastic Therapies

Chemotherapeutic agents and/or radiation and/or surgical removal of the neoplasm can optionally be combined with any of the methods of the present invention. Classes of compounds that can be used as the chemotherapeutic agent include: alkylating agents, antimetabolites, natural products and their derivatives, hormones and steroids (including synthetic analogs), and synthetics. Examples of alkylating agents (e.g., nitrogen mustards, ethylenimine derivatives, alkyl sulfonates, nitrosoureas and triazenes) include Uracil mustard, Chlormethine, Cyclophosphamide (Cytoxan^{®}), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide. Antimetabolites (including folic acid antagonists, pyrimidine analogs, purine analogs and adenosine deaminase inhibitors) may include, for example, Methotrexate, 5-Fluorouracil, Floxuridine, Cytarabine, 6-Mercaptopurine, 6-Thioguanine, Fludarabine phosphate, Pentostatine, and Gemcitabine. Natural products and their derivatives (including vinca alkaloids, antitumor antibiotics, enzymes, lymphokines and epipodophyllotoxins) may also be used and include, for example, Vinblastine, Vincristine, Vindesine, Bleomycin, Dactinomycin, Daunorubicin, Doxorubicin, Epirubicin, Idarubicin, paclitaxel (paclitaxel is commercially available as Taxol^{®}), Mithramycin, Deoxyco-formycin, Mitomycin-C, L-Asparaginase, Interferons (especially IFN-alpha), Etoposide, and Teniposide. Hormones and steroids (including synthetic analogs) include, for example, 17-alpha-Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estramustine, Medroxyprogesteroneacetate, Leuprolide, Flutamide, Toremifene, or Zoladex. Exemplary synthetics (including inorganic complexes such as platinum coordination complexes) include Cisplatin, Carboplatin, Hydroxyurea, Amsacrine, Procarbazine, Mitotane, Mitoxantrone, Levamisole, and Hexamethylmelamine.

Methods and dosages for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR), e.g., 1996 edition (Medical Economics Company, Montvale, N.J. 07645-1742, USA), the disclosure of which is incorporated herein by reference.

The following examples are provided for the purpose of illustrating the invention and should not be construed as limiting.

### Example 1

### Materials and Methods

### Producing Hybridomas

We immortalized spleen lymphocytes obtained from healthy donors by fusing them to the HAB-1 heteromyeloma as follows:
We washed the HAB-1 heteromyeloma cells twice with RPMI 1640 (PAA, Vienna, Austria) without additives and centrifuged the cells for 5 minutes at 1500 rpm. We then washed spleen lymphocytes twice with RPMI 1640 without additives and centrifuged these cells at 1500 rpm for 5 minutes. Both the HAB-1 and the lymphocyte cell pellets were resuspended in 10 ml RPMI 1640 without additives and were counted in a Neubauer cell counting chamber. We washed the cells again, added the HAB-1 cells and the lymphocytes together in a ratio of 1:2 to 1:3, mixed them, and centrifuged the mixture for 8 minutes at 1500 rpm. We pre-warmed Polyethylene Glycol 1500 (PEG) to 37°C and carefully let the PEG run drop-wise onto the pellet while slightly rotating the 50 ml tube. Next, we gently resuspended the pellet and rotated the tube for exactly 90 seconds in a 37°C water bath. We washed the cells twice with a full 10 ml pipette of RPMI-1640 without additives to remove the PEG and centrifuged the cells for 5 minutes at 1500 rpm. We added 1 ml of RPMI-1640 with HAT supplement (PAA, Vienna, Austria) and 10% Fetal Calf Serum (FCS), 1% glutamine, and 1% penicillin/streptomycin into each well of a 24-well plate. The cell pellet was dissolved in RPMI-1640 with HAT supplement and 1 x 10⁶ cells were added to each well of the 24-well plate. We then placed the 24-well plates into a humidified 37°C incubator and changed the RPMI 1640 medium with HAT supplement weekly. After four to six weeks, the cell culture supernatants were screened for antibody production in an enzyme-linked immunosorbent assay (ELISA).

In particular, the NORM-1 and NORM-2 human monoclonal antibodies were identified by using 2.5 x 10⁸ spleen lymphocytes derived from two healthy donors in a fusion experiment as described above. From this fusion, we obtained 181 clones out of a theoretical 250 clones, for a fusion frequency of 72%. Out of these 181 clones, the number of IgM producing clones was 40 (frequency = 22%), and out of these 40 clones, 9 expressed tumor-specific IgM antibodies (frequency = 22%).

### cDNA Synthesis and RT-PCR

To obtain the sequence of the NORM-1 and NORM-2 antibodies, we isolated whole RNA from the trioma using the RNASE Kit from Qiagen. Total RNA may also be prepared using methods standard in the art, e.g., those described in Krenn et al. (Clin. Exp. Immunol. 115:168-175, 1999). cDNA synthesis from total RNA obtained from hybridoma cell lines expressing NORM-1 and NORM-2 was performed with 5 µg total RNA using Gibco BRL (Eggenstein, Germany) M-MLV Reverse Transcriptase according to the manufacturer's instructions. The amplification of V_{H} and V_{L} genes was carried out in a 25 µl volume with 1.75 mM MgCl₂, 0.4 pM primer, 200 µM of each dNTP, and 1U Taq polymerase (MBI Fermentas, St. Leon-Rot, Germany). The PCR-products were amplified using the following cycle profiles: 95°C for 2 min, followed by 35 cycles of 94°C for 30 sec; 65°C for 30 sec (for VH3 and VH4 primers), 60°C for VH1, VH2, VH5, VH6 and 52°C for VL primers respectively; a final extension at 72°C for 4 min.

### Sequencing the Antibody

The PCR products were purified using gel electrophoresis through 2% agarose (Roth, Karlsruhe, Germany) followed by gel extraction of the PCR product using a JETSORB gel extraction kit (Genomed, Bad Oeynhausen, Germany). The PCR products were then cloned using the pCR-Script Amp SK⁺ cloning kit (Stratagene, Heidelberg, Germany). Ten positive clones were sequenced using the DyeDeoxy termination cycle sequencing kit (Applied BioSystems Inc., Weiterstadt, Germany) and analysed with an ABIPrism373 automated DNA sequencer. Both strands were sequenced using T3 and T7 primers. The sequences were analysed using the DNASIS for Windows sequence comparison software and the GenBank and IMGT/V-QUEST databases. The International Immunogenetics ("IMGT") database is coordinated by Marie-Paule Lefranc at the Université Montpellier, Montpellier, France.

### Immunohistochemical Staining of Paraffin Sections

Paraffin-embedded human tissues were sectioned (2 µm), deparaffinized and heated in citric acid (pH 5.5) in a pressure cooker for 5 minutes. The sections were blocked with bovine serum albumin (BSA 5 mg/ml) diluted in phosphate buffered saline (PBS) for 30 minutes at room temperature. Treated sections were then incubated either with the different IgM antibodies (10 µg/ml) or with positive control antibodies (anti-cytokeratin 8 antibody or anti-cytokeratin 7 antibody, Dako, Hamburg, Germany, diluted 1:20 with BSA/PBS) for 2.5 hours at 37°C in a humidified incubator. The sections were then washed three times with Tris/NaCl (3 grams Tris, 40.5 grams NaCl in 5 litres of distilled H₂O and pH adjusted to 7.4 with HCl), followed by incubation with peroxidase-labeled rabbit anti-human IgM antibody diluted 1:50 in PBS containing 30% rabbit serum (for human IgM antibodies) or rabbit anti-mouse conjugate (Dako, Hamburg, Germany) diluted 1:50 in PBS containing 30% human AB plasma (for positive control antibodies) at room temperature for 1 hour. After washing three times with Tris/NaCl, the tissue sections were incubated in PBS for 10 min before staining with diaminobenzidine (0.05%)-hydrogen peroxide (0.02%) (Sigma, Taufkirchen (München), Germany) for another 10 minutes at room temperature. The reaction was stopped under running tap water, and the sections were counterstained with hematoxylin. After mounting with glycerol-gelatin, the sections were analyzed using light microscopy.

### Immunohistochemical Staining of Cryo-Sections from Tumors

Frozen human tissues were sectioned (4 µm), fixed in acetone, air-dried and washed with Tris/NaCl (3 grams Tris, 40.5 grams NaCI in 5 litres of distilled H₂O and pH adjusted to 7.4 with HCl). The cryo-sections were then blocked with PBS containing 3% milk powder for 30 minutes at room temperature. After washing three times with Tris/NaCl the sections were incubated with NORM-1 or NORM-2 human IgM antibodies or unrelated human monoclonal IgM (Chrompure IgM, Dianova) at the same concentration or mouse anti-cytolceratin 8 antibody diluted 1:50 with BSA/PBS (Dako, Hamburg, Germany) for 30 minutes at room temperature. The sections were washed three times with Tris/NaCl, followed by incubation with secondary antibodies (peroxidase-labeled rabbit anti-human or rabbit anti-mouse conjugate 1:50) for 30 minutes at room temperature. After washing three times with Tris/NaCl and incubation in PBS for 10 minutes, the sections were stained with diaminobenzidine (0.05%)-hydrogen peroxide (0.02%) (Sigma, Taufkirchen (Munchen), Germany) for 10 minutes at room temperature. The reaction was stopped under running tap water and the sections counterstained with hematoxylin. After mounting with glycerol-gelatin, the sections were analyzed using light microscopy.

### Cytospin Preparation

The adherent growing cells were detached by adding Trypsin/EDTA (PAA, Vienna, Austria) followed by a 5 minute incubation in an humidified incubator (37°C, 5% CO₂) and centrifugation for 5 minutes at 1,500 rpm. The cells then were washed twice with 10ml of RPMI-1640 cell culture medium (PAA, Vienna, Austria). The cell number was adjusted to a density of 1 x 10⁵ cells/ml. From this solution, 100µl were centrifuged onto microscope slides with a cytospin centrifuge (CYTOSPIN 2, Shandon, UK) for 2 minutes at 50 rpm. The resultant cytospins were dried for at least 2 hours and stained as specified below.

### Immunoperoxidase Staining of Cytospins

Cytospins were dried for at least two hours at room temperature. The cytospins were then fixed for 10 minutes in acetone. The fixed cytospins were dried for 30 minutes at room temperature, washed three times with Tris-NaCl (3 grams Tris, 40.5 grams NaCl in 5 litres of distilled H₂O and pH adjusted to 7.4 with HCl), and placed into Tris/NaCl for 5 minutes. The cytospins were blocked for 15-30 minutes with 3% milk powder in PBS (100 µl per cytospin) and washed three times with Tris-NaCl. The cytospins were incubated in 100µl of primary antibody per cytospin (e.g., at 20 µg/ml in 0.5% BSA/PBS; anti-cytokeratin 8 at 1:50 in BSA/PBS; or RPMI 1640 media (PAA, Vienna, Austria) as a negative control) for 30 minutes in a humidified chamber at room temperature. Following the incubation, the cytospins were washed three times with Tris-NaCl.

The cytospins were then incubated in 100µl of a solution containing the secondary antibody (70% PBS + 30% rabbit or human serum + e.g., 1:50 rabbit anti-mouse antibody, peroxidase-coupled or 1:50 rabbit anti-human IgM antibody, peroxidase-coupled; Dako, Hamburg, Germany) per cytospin for 30 minutes in a humidified chamber at room temperature and washed three times with Tris-NaCl and placed into PBS for 10 minutes. The cytospins where then incubated for 10 minutes in 100 µl of a solution containing 0.05% diaminobenzidine and 0.02% hydrogen peroxide (Sigma, Taufkirchen (Munchen), Germany). Following the incubation, the cytospins were washed with distilled H₂O and placed into a hematoxylin staining solution (Roth, Karlsruhe, Germany) for 5 minutes. The cytospins were then rinsed for 15 minutes under running tap water, washed with distilled H₂O, and cover with pre-warmed glycerol-gelatin.

The following experiments were carried out using the above materials and methods.

### Example 2

### Generation of the Cell Line Expressing the NORM- 1 or NORM-2 Monoclonal Antibody

As described above, we obtained the NORM-1 and NORM-2 monoclonal antibody-expressing hybridoma by fusing lymphocytes obtained from the spleen of a healthy donor with a heteromyeloma cell line. The resultant cell is a type of hybridoma known as a trioma, as it is the fusion of three cells. Like normal B-lymphocytes, this trioma has to ability to produce antibodies. The specificity of the antibody is determined by the specificity of the original lymphocyte from the patient that was used to generate the trioma.

In particular, single cell suspensions were prepared by mechanical disruption of the spleen followed by either immediate fusion or cryo-preservation. For immortalization, 2.5 x 10⁸ spleen lymphocytes derived from healthy donors were incubated with heteromyeloma HAB-1 cells (Faller, et al., Br. J. Cancer 62:595-598, 1990) and fusion of the lymphocytes and HAB-1 cells was facilitated using polyethylene glycol 1500 (Roche, Mannheim, Germany). Hybridomas were seeded in 24-well plates and cultured in RPMI-1640 (PAA, Vienna, Austria) containing 10% Fetal Calf Serum (FCS) and 10% HAT (hypoxanthine-aminopterin-thymidine) supplement (PAA, Vienna, Austria), 1% Glutamine, and 1% Penicillin/Streptomycin. After about four weeks, the supernatants of the hybridomas were screened for antibody content using an ELISA. We obtained 40 IgM producing clones. ELISA positive clones were then tested immunohistochemically on a panel of different tumor tissues and normal tissues to verify tumor specificity. Of the 40 IgM producing clones, 9 produced tumor-specific IgM antibodies. Tumor-specific antibodies were further characterized immunohistochemically, genetically, biochemically, and using molecular biology methods standard in the art.

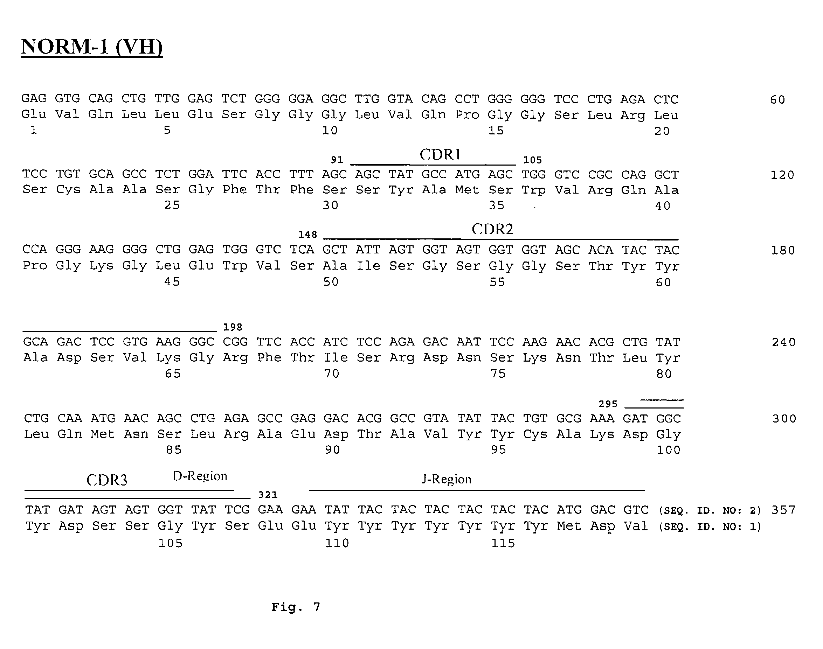

The amino acid sequence (SEQ ID NO:1) and the nucleic acid sequence (SEQ ID NO:2) of the variable region of the heavy chain of human monoclonal antibody NORM-1 are shown in Figure 7. As indicated in Figure 7, Complement Determining Region 1 (CDR1) of the NORM-1 variable region heavy chain spans nucleotides 91-105 which encode amino acids 31-35, CDR2 spans nucleotides 148-198 which encode amino acids 50-66, and CDR 3 spans nucleotides 295-321 which encode amino acids 99-107. In addition, the D-region spans nucleotides 297-319 and the J-region spans nucleotides 327-357.

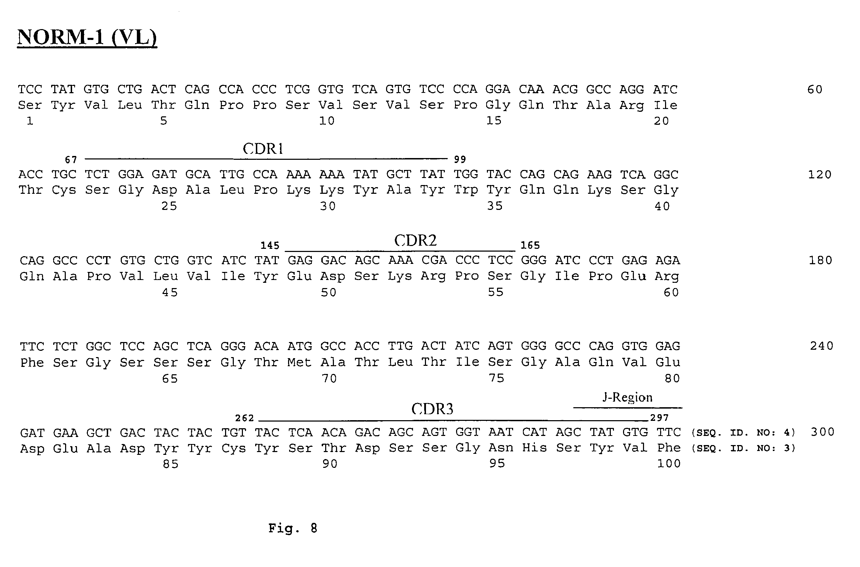

The amino acid sequence (SEQ ID NO:3) and the nucleic acid sequence (SEQ ID NO:4) of the variable region of the light chain of human monoclonal antibody NORM-1 are shown in Figure 8. As indicated in Figure 8, CDR1 of the NORM-1 variable region light chain spans nucleotides 67-99 which encode amino acids 23-33, CDR2 spans nucleotides 145-165 which encode amino acids 49-55, and CDR3 spans nucleotides 262-297 which encode amino acids 88-99. In addition, the J-region spans nucleotides 291-300.

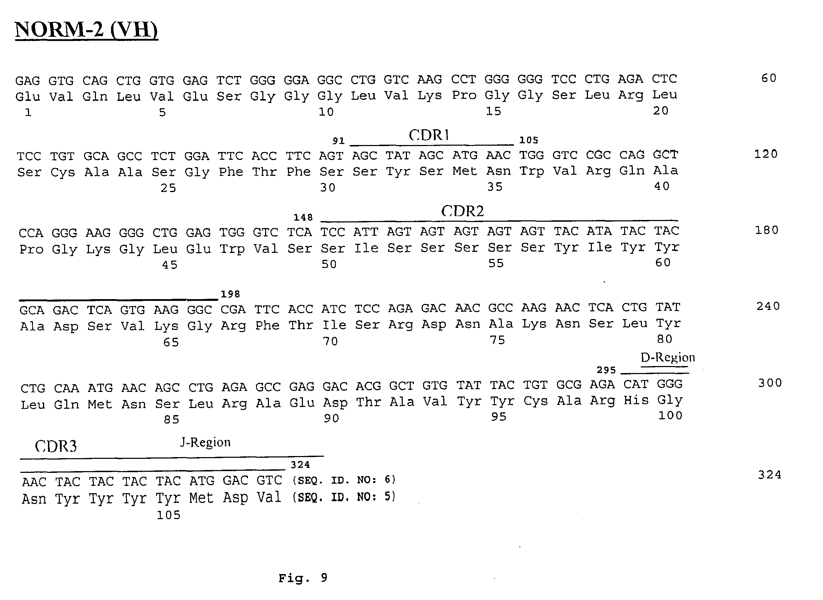

The amino acid sequence (SEQ ID NO:5) and the nucleic acid sequence (SEQ ID NO:6) of the variable region of the heavy chain of human monoclonal antibody NORM-2 are shown in Figure 9. As indicated in Figure 9, CDR1 of the NORM-2 variable region heavy chain spans nucleotides 91-105 which encode amino acids 31-35, CDR2 spans nucleotides 148-198 which encode amino acids 50-66, and CDR3 spans nucleotides 295-324 which encode amino acids 99-108. In addition, the D-region spans nucleotides 297-300 and the J-region spans nucleotides 301-324.

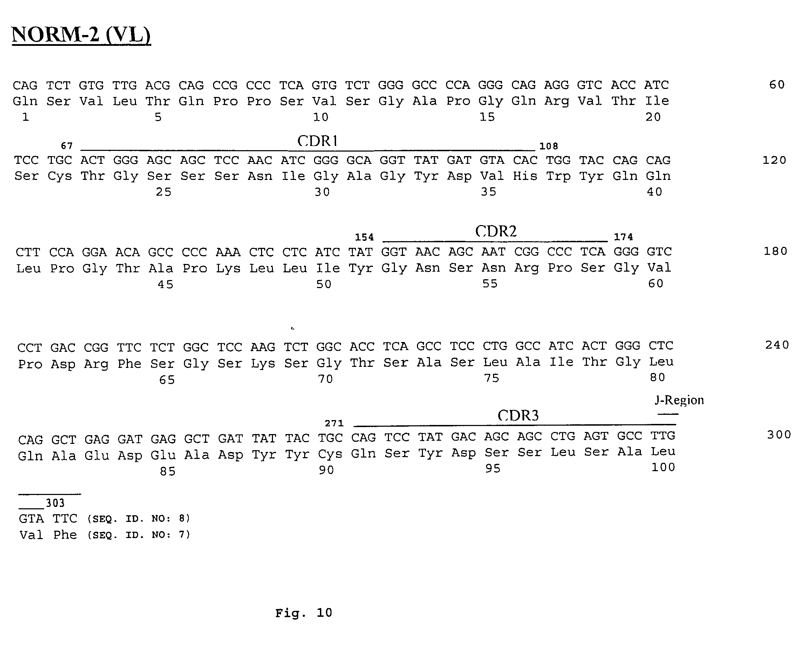

The amino acid sequence (SEQ ID NO:7) and the nucleic acid sequence (SEQ ID NO:8) of the variable region of the light chain of human monoclonal antibody NORM-2 are shown in Figure 10. As indicated in Figure 10, CDR1 of the NORM-2 variable region light chain spans nucleotides 67-108 which encode amino acids 23-36, CDR2 spans nucleotides 154-174 which encode amino acids 52-58, and CDR3 spans nucleotides 271-303, which encode amino acids 91-101. In addition, the J-region spans nucleotides 299-306.

### Example 3

### Immunohistochemical Characterization of an Antibody

To investigate the genetic origin of the NORM-1 and NORM-2 human monoclonal IgM antibodies the V_{H} and V_{L} genes were amplified, cloned and sequenced. The sequences were compared with germ-line sequences in the IMGT/V-QUEST database to identify the most homologous germ-line genes and to detect somatic mutations. The results are represented in Table 1. The degree of identity of the nucleotide sequences of the V_{H} segment to those of the closest reported germ-line VH genes was 100%. The antibodies contain V_{H} region encoded by the V_{H}3 gene family. The close homology of the V_{H} regions to the germ-line genes and the low R/S ratio indicate that NORM-1 and NORM-2 antibodies did not undergo affinity maturation by somatic mutation due to antigen contact. The degree of identity of the nucleotide sequences of the V_{L} segment to their most homologous V_{L} germ-line genes ranged from 99.3 to 99.6%, with both antibodies utilizing λ-light chain genes. The R/S ratio was low again, and the mutations were restricted to the framework region.

**Table 1: Characterization of Variable Heavy and Light Chain Regions of the NORM-1 and NORM-2 Monoclonal IgM Antibodies.**

| **Heavy Chain** | | | | | |
|---|---|---|---|---|---|
| Antibody | V_{H}-Family | Germ-Line Gene | Homology | R/S | R/S |
| | | | (%) | Frame | CDR |
| NORM-1 | V_{H}3 | DP-47, (lgHV3-23*01) | 100 | 0/0 | 0/0 |
| NORM-2 | V_{H}3 | DP-77, (lgHV3-21*01) | 100 | 0/0 | 0/0 |
| | | | | | |

| **Light Chain** | | | | | |
|---|---|---|---|---|---|
| Antibody | V_{L}-Family | Germ-Line Gene | Homology | R/S | R/S |
| | | | (%) | Frame | CDR |
| NORM-1 | λ | IgLV3-10*01 | 99.3 | 1/1 | 0/0 |
| NORM-2 | λ | IgLV1-40*01 | 99.6 | 1/0 | 0/0 |

After initial testing on different tumor tissues, the reaction patterns of the antibodies were investigated in greater detail using immunohistochemical staining on a variety of paraffin- and cryo-embedded carcinomas and normal tissues. The NORM-1 and NORM-2 antibodies exhibited no binding activity with normal tissues (Table 2).

**Table 2: Reaction Pattern of the NORM-1 and NORM-2 Monoclonal IgM Antibodies on Normal Tissues**

| **Tissue** | **NORM-1** | **NORM-2** |
|---|---|---|
| Colon | - | - |
| Stomach | - | - |
| Pancreas | - | - |
| Lung | - | - |
| Esophagus | - | - |
| Breast | - | - |
| Uterus | - | - |

In contrast, the NORM-1 and NORM-2 antibodies specifically stain a number of tumor tissues (see Table 3).

**Table 3: Reaction Pattern of the NORM-1 and NORM-2 Monoclonal IgM Antibodies on Tumor Tissues.**

| Tumor Type | NORM-1 | NORM-2 |
|---|---|---|
| Colon Adenocarcinoma | + | + |
| Stomach Diffuse-Type Adenocarcinoma | + | + |
| Pancreas Adenocarcinoma | + | + |
| Lung Adenocarcinoma | + | + |

Figures 1 and 2 show examples of the reactivity patterns of the NORM-1 and NORM-2 antibodies on several tumor tissues in comparison with staining patterns on normal tissue of the same organ. As is indicated in these figures, the NORM-1 and NORM-2 antibodies isolated from healthy donors specifically stain tumor cells, while the surrounding tissue and normal tissue are not stained.

Moreover, the NORM-1 and NORM-2 monoclonal antibodies also specifically stain a number of carcinoma cell lines. In particular, the NORM-1 antibody stains lung squamous cell carcinoma cell line EPLC-272H (DSMZ Accession Number ACC 383); lung adeno carcinoma cell line Colo-699 (DSMZ Accession Number ACC 196); Colon carcinoma cell line CACO-2 (DSMZ Accession Number ACC 169, ATCC Accession Number HTB-37); Colon carcinoma cell line Colo-206F (DSMZ Accession Number ACC 21); stomach carcinoma cell line 23132/87 (DSMZ Accession Number ACC 201); pancreas carcinoma cell line ASPC-1 (ATCC Accession Number CRL-1682); prostate carcinoma cell line DU-145 (DSMZ Accession Number ACC 261, ATCC Accession Number HTB-81); and prostate carcinoma cell line BM1604 (DSMZ Accession Number ACC 298). The NORM-2 antibody stains Colo-699; CACO-2; 23132/87; DU-145; and BM1604 cells. Slides of these cells were stained according to the cytospin protocol described in the materials and methods section.

### Example 4

### Determining whether an Antibody Inhibits Cell Proliferation

Cell proliferation may be assayed by a number of methods that are standard in the art, for example, by the reduction of tetrazolium salts. The yellow tetrazolium salt 3-(4,5-dimethylthiazol-2-yl) - 2,5-diphenyltetrazolium bromide ("MTT") (Sigma, St. Louis, MO), is reduced by metabolically active cells, in part by the action of mitochondrial dehydogenase enzymes to generate reducing equivalents such as NADH and NADPH. The resulting intracellular purple formazan can be solubilized and quantified by spectrophotometric means. The MTT cell proliferation assay measures the rate of cell proliferation and, when metabolic events lead to apoptosis, the reduction in cell viability.

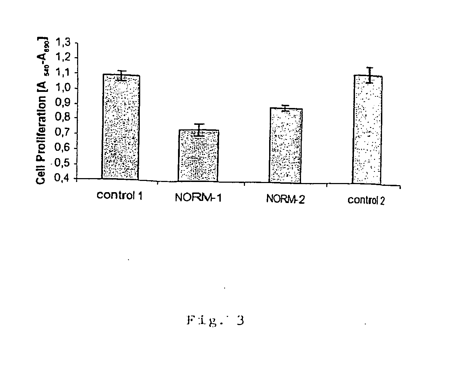

For the MTT assay, we used the human stomach adenocarcinoma cell line 23132/87. This carcinoma cell line was derived from a freshly prepared primary culture of a gastric tumor patient and, in general, we used early passages of this cell line (less than ten) to avoid cell culture artifacts. We trypsinized 23132/87 cells and diluted them to 1 × 10⁶ cells/ml in complete growth medium. 50 µl of this suspension were pipetted into wells of a 96-well plate, resulting in approximately 5 × 10⁴ cells/well. The first row of wells was left empty. We then added 50 µl of the antibody diluted in complete medium to each well. To demonstrate normal growth, the cells were supplemented with complete growth medium (Control 1). An unrelated human IgM antibody (Chrompure IgM, Dianova) at the same concentration served as a negative control (Control 2). The 96-well plate was then incubated for 48 hours in a humidified 37°C incubator. After the incubation period, 50 µl MTT solution (5 mg/ml in PBS) were added to each well. The 96-well plate was incubated for 30 minutes at 37°C and centrifuged for 5 minutes at 800 x g. The supernatant was aspirated, 150 µl of dimethylsulphoxide (DMSO) were added to each well, and the cell pellet was resuspended.
Absorption was determined at a wavelength of 540 nm and at a reference wavelength of 690 nm in an ELISA reader. After 48 hours, the NORM-1 and NORM-2 antibodies inhibited cell proliferation of 23132/87 stomach carcinoma cells relative to the controls (Fig. 3).

### Example 5

### Determining whether an Antibody Induces Apoptosis

A number of assays standard in the art may be used to determine if an antibody induces apoptosis of a cell.

For example, we used the CELL DEATH DETECTION ELISA^{PLUS} (Roche, Mannheim, Germany) to analyze the extent to which the NORM-1 and NORM-2 antibodies induce apoptosis. The cell death detection ELISA is based on a quantitative sandwich-enzyme-immunoassay principle using mouse monoclonal antibodies directed against DNA and histones, respectively. This assay allows the specific determination of mono- and oligo-nucleosomes that are released into the cytoplasm of cells that die from apoptosis.

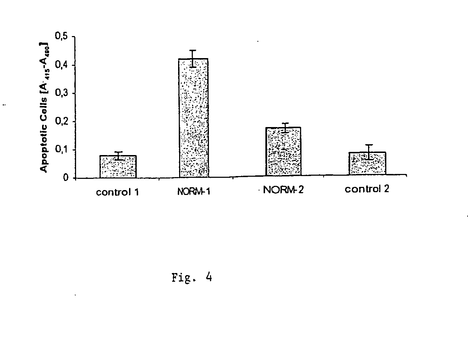

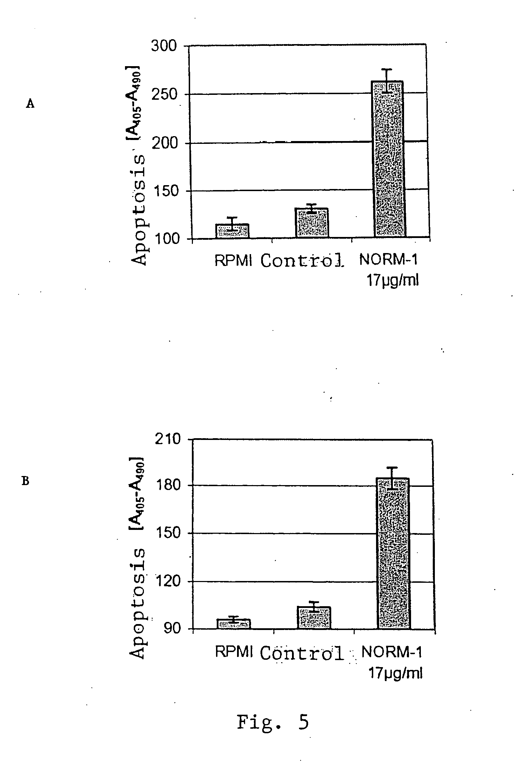

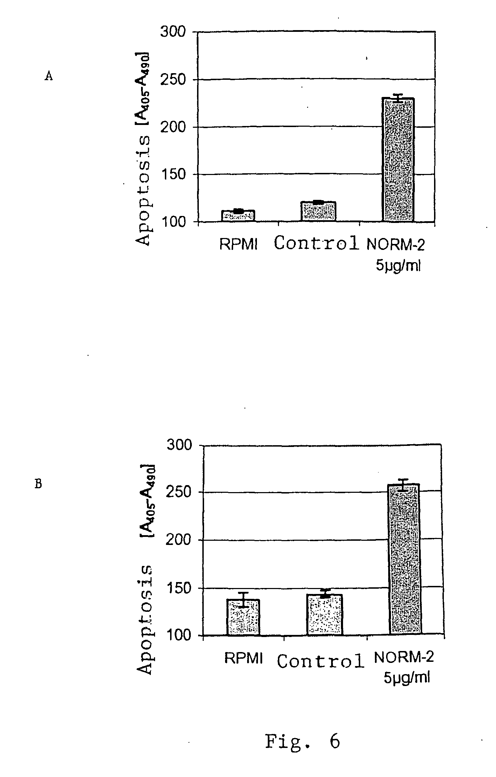

In particular, 1 x 10⁴ 23132/87 stomach carcinoma cells were plated on 96-well plates and incubated in presence of different concentrations of the NORM-1 and NORM-2 antibodies for 48 hours at 37°C and 7% CO₂ in a humidified CO₂ incubator. To demonstrate normal growth, the cells were supplemented with complete growth medium (Control 1; RPMI). An unrelated human IgM antibody (Chrompure IgM, Dianova) at the same concentration served as a negative control (Control 2). After the incubation period, the cells were centrifuged for 10 minutes at 200g and the supernatants were removed. The resulting cell pellets were then incubated with lysis-buffer for 30 minutes at room temperature. After centrifugation the supernatants were transferred into a streptavidin-coated microtiter plate (MTP) and immunoreagent (a mixture of 10% Anti-Histone-Biotin, 10% Anti-DNA-peroxidase (Anti-DNA POD) and 80% incubation buffer) added before incubation for 2 hours at room temperature on a MTP shaker at 250 rpm. Following the incubation period, unbound components were removed by a washing step with incubation buffer. Peroxidase activity was determined photometrically with ABTS™ as a substrate (1 ABTS™ (2,2'-Azino-di[3-ethyl-benz-thiazolin-sufonat) tablet in 5 ml substrate buffer). Antibody-induced apoptosis was measured by determining the color intensity of the green precipitate that it formed as a result of this reaction using an ELISA reader at a wavelength of 415nm or 405nm in comparison to ABTS™ solution as a blank (reference wavelength of approximately 490 nm). Based on this color intensity, we calculated the level of the antibody-induced apoptosis. These experiments clearly demonstrated that each antibody, NORM-1 and NORM-2, induces apoptosis in carcinoma cells after 24 and 48 hours of incubation when compared to media controls and control antibodies (Fig. 4, Fig. 5A and 5B, Fig. 6A and 6B).

### Example 6

### In Vivo Imaging of a Neoplasm

A patient suspected of having a neoplasm, such as a colorectal carcinoma, may be given a dose of radioiodinated NORM-1 or NORM-2 antibody, or another tumor-specific polypeptide, and radiolabeled unspecific antibody using the methods described herein. Localization of the tumor for imaging may be effected according to the procedure of Goldenberg et al. (N. Engl. J. Med., 298:1384, 1978). By I.V. an infusion of equal volumes of solutions of ¹³¹I-NORM-1 or NORM-2 antibody and Tc-99m-labeled unspecific antibody may be administered to a patient. Prior to administration of the reagents I.V., the patient is typically pre-tested for hypersensitivity to the antibody preparation (unlabeled) or to antibody of the same species as the antibody preparation.
To block thyroid uptake of ¹³¹I, Lugol's solution is administered orally, beginning one or more days before injection of the radioiodinated antibody, at a dose of 5 drops twice or three-times daily. Images of various body regions and views may be taken at 4, 8, and 24 hours after injection of the labeled preparations. If present, the neoplasm, e.g., a stomach adenocarcinoma, is detected by gamma camera imaging with subtraction of the Tc-99m counts from those of ¹³¹I, as described for ¹³¹I -labeled anti-CEA antibody and Tc- 99m-labeled human serum albumin by DeLand et al. (Cancer Res. 40:3046, 1980). At 8 hours after injection, imaging is usually clear and improves with time up to the 24 hour scans.

### Example 7

### Treatment of a Neoplasm Using Labeled Antibody Mixtures

A patient diagnosed with a neoplasm, for example, a patient diagnosed with a stomach adenocarcinoma, may be treated with polypeptides identified using the methods of the invention as follows. Lugol's solution may be administered, e.g., 7 drops 3 times daily, to the patient. Subsequently, a therapeutic dose of ¹³¹I-NORM-1 or NORM-2 antibody may be administered to the patient. For example, a ¹³¹I dose of 50 mCi may be given weekly for 3 weeks, and then repeated at intervals adjusted on an individual basis, e.g., every three months, until hematological toxicity interrupts the therapy. The exact treatment regimen is generally determined by the attending physician or person supervising the treatment. The radioiodinated antibodies may be administered as slow I.V. infusions in 50 ml of sterile physiological saline. After the third injection dose, a reduction in the size of the primary tumor and metastases may be noted, particularly after the second therapy cycle, or 10 weeks after onset of therapy.

### Example 8

### Treatment Using Conjugated Antibodies

A patient diagnosed with a neoplasm, for example, a patient with stomach cancer that has metastasized, may be treated with solutions of ¹³¹I-NORM-1 or NORM-2, ¹⁰B-NORM-1 or NORM-2, and a Tc-99m labeled unspecific antibody. An amount of ¹³¹I-labeled NORM-1 or NORM-2 antibody (in 50 ml of sterile physiological saline) sufficient to provide 100 mCi of ¹³¹I activity based on a 70 kg patient weight may be administered to the patient. This dosage is equal to 3.3 mg of an antibody having 40-80 Boron atoms and 8-16 Boron-10 atoms per antibody molecule. The neoplasm is first precisely localized using the procedure of Example 6. In addition, Lugol's solution should be continuously administered to the patient, as in the previous example. A well-collimated beam of thermal neutrons may then be focused on the defined tumor locations. Irradiation with an external neutron beam dose of 400-800 rads, delivered in a period of from 8-20 min, is effected for each tumor locus, and is optionally repeated with administration of the tumor-locating antibody, with or without the radiolabel, at intervals adjusted on an individual basis, but usually not exceeding a total dose of 3200 rads unless simultaneous external irradiation therapy is indicated. If desired, in addition to this therapy, an anti-tumor agent, such as a chemotherapeutic agent, may also be administered to the patient.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure come within known or customary practice within the art to which the invention pertains and may be applied to the essential features hereinbefore set forth.

International Patent Application Nos. PCT/IB03/0133 5 and PCT/IB03/03487, U.S. Patent Nos. 5,367,060 and 5,641,869, and all other references cited herein are hereby incorporated by reference.

## Claims

1. (New) A purified or isolated antibody or a functional fragment thereof comprising:
(a) a heavy chain variable region with at least 75% identity to the amino acid sequence of SEQ ID NO:5 and a light chain variable region with at least 75% identity to the amino acid sequence of SEQ ID NO: 7; or
(b) a heavy chain variable region with at least 75% identity to the amino acid sequence of SEQ ID NO:1 and a light chain variable region with at least 75% identity to the amino acid sequence of SEQ ID NO:3,
wherein the antibody or functional fragment thereof specifically binds to an adenocarcinoma of the colon, a diffuse-type stomach carcinoma, an adenocarcinoma of the pancreas, or an adenocarcinoma of the lung.

2. (New) A purified or isolated antibody or a functional fragment thereof comprising:
(a) a heavy chain variable region with at least 75% identity to the amino acid sequence of SEQ ID NO:5 and a light chain variable region with at least 75% identity to the amino acid sequence of SEQ ID NO:7; or
(b) a heavy chain variable region with at least 75% identity to the amino acid sequence of SEQ ID NO:1 and a light chain variable region with at least 75% identity to the amino acid sequence of SEQ ID NO:3,
wherein the antibody or functional fragment thereof specifically binds to Colo-699 (DSMZ Accession Number ACC 196), CACO-2 (DSMZ Accession Number ACC169, ATCC Accession Number HTB-37), 23132/87 (DSMZ Accession Number ACC 201), DU-145 (DSMZ Accession Number ACC 261, ATCC Accession Number HTB-81), or BM 1604 (DSMZ Accession Number ACC 298) cells.

3. (New) The purified or isolated antibody or a functional fragment thereof of claims 1 or 2, wherein said antibody or a functional fragment thereof comprises a heavy or a light chain variable region with at least 80% identity to the amino acid sequence of SEQ ID NO:5, or SEQ ID NO:7, or SEQ ID NO:1, or SEQ ID NO:3.

4. (New) The purified or isolated antibody or a functional fragment thereof of claims 1 or 2, wherein said antibody or a functional fragment thereof comprises a heavy or a light chain variable region with at least 85% identity to the amino acid sequence of SEQ ID NO:5, or SEQ ID NO:7, or SEQ ID NO:1, or SEQ ID NO:3.

5. (New) The purified or isolated antibody or functional fragment of claims 1 or 2, wherein said antibody or functional fragment thereof comprises amino acids 31-35, 50-66, and 99-108 of SEQ ID NO:5, or amino acids 23-36, 52-58, and 91-101 of SEQ ID NO:7.

6. (New) The purified or isolated antibody or functional fragment of claims 1 or 2, wherein said antibody or functional fragment thereof comprises amino acids 31-35, 50-66, and 99-107 of SEQ ID NO:1, or amino acids 23-36, 49-55, and 88-99 of SEQ ID NO:3.

7. (New) The purified or isolated antibody or functional fragment of any of claims 1 to 6, wherein said antibody is a monoclonal antibody or a functional fragment thereof.

8. (New) The purified or isolated antibody or functional fragment of any of claims 1 to 7, wherein said functional fragment is selected from the group consisting of V_{L}, V_{H}, Fᵥ, Fab, Fab', and F(ab')₂.

9. (New) The purified or isolated antibody or functional fragment of any of claims 1 to 8, wherein the heavy or light chain variable region has an insertion, deletion or substitution of an amino acid residue in SEQ ID NO:1, SEQ ID NO:3, SEQ ID NO:5 or SEQ ID NO:7.

10. (New) The purified or isolated antibody or functional fragment of any of claims 1 to 9, wherein said antibody or functional fragment induces apoptosis of any one of Colo-699 (DSMZ Accession Number ACC 196), CACO-2 (DSMZ Accession Number ACC169, ATCC Accession Number HTB-37), 23132/87 (DSMZ Accession Number ACC 201), DU-145 (DSMZ Accession Number ACC 261, ATCC Accession Number HTB-81), or BM 1604 (DSMZ Accession Number ACC 298) cells.

11. (New) The purified or isolated antibody or functional fragment of any of claims 1 to 9, wherein said antibody or functional fragment decreases proliferation of any one of Colo-699 (DSMZ Accession Number ACC 196), CACO-2 (DSMZ Accession Number ACC169, ATCC Accession Number HTB-37), 23132/87 (DSMZ Accession Number ACC 201), DU-145 (DSMZ Accession Number ACC 261, ATCC Accession Number HTB-81), or BM 1604 (DSMZ Accession Number ACC 298) cells.

12. (New) A nucleic acid that encodes the purified or isolated antibody or functional fragment of any of claims 1 to 11.

13. (New) A vector comprising the nucleic acid of claim 12.

14. (New) An isolated cell that that expresses the antibody or functional fragment of any of claims 1 to 11.

15. (New) A medicament comprising the purified or isolated antibody or functional fragment of any of claims 1 to 11 in a pharmaceutically acceptable carrier.

16. (New) Use of the purified antibody or antigen binding fragment thereof of any one of claims 1 to 11 in a method of diagnosing a neoplasm in a mammal, said method comprising the steps of:
(a) contacting a cell or tissue sample of said mammal with the purified antibody or antigen binding fragment thereof of any one of claims 1 to 11, and
(b) detecting whether said purified antibody or antigen binding fragment thereof binds to said cell or tissue sample, wherein binding of said purified antibody or antigen binding fragment thereof to said cell or tissue sample is indicative of said mammal having a neoplasm.

17. (New) Use of the purified antibody or antigen binding fragment thereof of any one of claims 1 to 11 in the manufacture of a medicament for treating a proliferative disorder in a mammal, said use comprising contacting a cell or tissue with the purified antibody or antigen binding fragment thereof of any one of claims 1 to 11, wherein binding of said purified antibody or antigen binding fragment thereof to said cell or tissue sample results in the induction of apoptosis of said cell or tissue.

18. (New) The use of claims 16 or 17, wherein said antibody or antigen binding fragment thereof is conjugated to a detectable agent selected from the group consisting of a radionuclide, a fluorescent marker, an enzyme, a cytotoxin, a cytokine, and a growth inhibitor.

19. (New) The use of claims 16 or 17, wherein said mammal is a human.

20. (New) A method of generating an antibody or functional fragment of any of claims 1 to 11, comprising expressing nucleic acid sequences encoding the antibody or functional fragment of any of claims 1 to 11 in a host cell.
